# FASCICULE DE BREVET EUROPEEN

(11) **EP 0 532 370 B1**
(45) Date de publication et mention de la délivrance du brevet: **20.05.1998**
(21) Numéro de dépôt: 92402076.1
(22) Date de dépôt: 17.07.1992
(51) Int. Cl.: C07H 15/04, B01F 17/00, C11D 1/66, A61K 7/08, C09K 19/06, A23L 1/03, C07H 7/033

(54) **Dérivés de l'acide galacturonique, leurs procédés de préparation et leurs applications**
Galakturonsäurederivate, Verfahren zur ihrer Herstellung und ihre Verwendung
Galacturonic acid derivatives, process for their preparation and their uses

(30) Priorité: 22.07.1991 FR 9109226
(43) Date de publication de la demande: 17.03.1993
(60) Demande divisionnaire: 94201064.6
(73) Titulaire: AGRO INDUSTRIE RECHERCHES ET DEVELOPPEMENTS (A.R.D.), F-75008 Paris (FR); ZSCHIMMER & SCHWARZ GmbH & Co. CHEMISCHE FABRIKEN, D-56108 Lahnstein (DE)
(72) Inventeur: Petit, Serge, F-60150 Montmacq (FR); Ralainirina, Robert, F-80080 Amiens (FR); Favre, Serge, F-60200 Compiegne (FR); Baynast, Régis de, F-78000 Versailles (FR)
(74) Mandataire: Bourgognon, Jean-Marie

(56) Documents cités:
- EP-A- 0 326 673
- EP-A- 0 334 498
- EP-A- 0 427 210
- US-A- 3 873 614
- TENSIDE, SURFACTANTS, DETERGENTS vol. 26, 1989, pages 318 - 324 TH. BOECKER ET AL 'Synthese und Eigenschaften von Kohlenhydrattensiden'
- CHEMICAL ABSTRACTS, vol. 109, 1988, Columbus, Ohio, US; abstract no. 190679t, Y. HUI ET AL. 'Synthesis of glucoside surfactants and asymmetric reduction of phenyl alkyl ketones in chiral micelles'
- LIFE SCIENCES vol. 50, 1992, pages 1773 - 1779 Y. NAMBA ET AL 'Liposomal modification with uronate, which endows liposomes with long circulation in vivo, reduces the uptake of liposomes by J744 cells in vitro'

## Description

La présente invention concerne des dérivés de l'acide galacturonique, leurs procédés de préparation et leurs diverses applications, notamment comme agents tensio-actifs non ioniques ou anioniques.

Le greffage de groupes alkyle sur des glucides conduit à des agents tensio-actifs dont les propriétés de surface sont très intéressantes et dont la biodégradabilité est généralement bonne (R.D. SWISHER, "Surfactant biodégradation", Marcel Dekker, Inc. NEW YORK, 1987).

Les matières premières le plus souvent utilisées sont le saccharose et le glucose et les réactions effectuées sur ces substrats non protégés conduisent à des mélanges complexes de tensio-actifs non ioniques, ceci étant dû à des greffages statistiques (mono, di, triaddition) et à des phénomènes d'oligomérisation inter et/ou intra moléculaires (voir EP 0249013, DE 3842141, DE 3723826, H. LUDERS et P. HOFFMANN "Synthesis, Chemical Structure and Properties of Alkylpolyglucosides", Césio, 2ème Congrès Mondial sur les Surfactants, 24-27 mai 1988, PARIS, et, D. BALZET "Alkylpolyglucosides, their physico-chemical properties and their uses", Tenside Surf. Det. 28. 1991, 6). Les températures de réaction utilisées dans ces préparations sont généralement élevées (supérieures à 100 °C), ce qui entraîne une dégradation partielle des substrats et provoque une coloration des produits.

Pour ce qui est des tensio-actifs anioniques à base de glucides, les rares composés décrits dans la littérature sont obtenus par bioconversion (sophoroses lipides, rhamnolipides... : voir DE 352 6417 ; G. Georgiu, S.C. Lin et M.M. Sharma, "Surface active compounds from microorganisms", Bio./Biotechnology, vol 10, January 1992, 60-65 ; D.F. Gerson et J.E. Zajic "microbial biosurfactants, Process Biochemistry, July 1979, 20-29 ; D.G. Cooper "Biosurfactants", Microbiological Sciences", vol 3, n°5, 1986, 145-149) ou par oxydation de l'hydroxyle primaire de D-octyl-glucopyranoside, de l'α-D-dodécylglucopyranoside, de l'α-D-tetradécyl glucopyranoside du β-D-décyl maltoside, de l'α-D-tetradécyl maltoside (voir EP 0326673 ; Bocker J. Thiem J., Tenside, Surf, Det., 26 318, (1989) ; Van Bekkum H.dans "Carbohydrates as organic raw materials" Lichtenthaler F.W. (Ed), V.C.H. Weinheim 289 (1991) et références citées: de GOEDE A.T.J.W., de Wit P., Vinki P. Van Rantwijk F. et Van Bekkum H, présentation au 6ème Congrès Européen sur la chimie des carbohydrates, Edimbourg, 1991), soit par voie catalytique, soit par voie chimique avec le complexe trioxyde de soufre/pyridine [voir Miam. H, Anderson C.E. et Kent P.W. Biochem. J., 181, 387,(1979)] pour obtenir respectivement les composés carboxylés ou sulfatés correspondants. Ces documents ne décrivent pas de dérivés du galactose, ni bien sûr leurs propriétés, malgré des généralisations hasardeuses. De plus, les substrats nécessaires à ces synthèses sont difficiles à obtenir, et les seuls protocoles conduisant à des composés de structure bien définie nécessitent toujours une protection préalable, utilisent des solvants organiques et des réactifs coûteux (carbonate d'argent) ; c'est le cas notamment de l'octyl glucoside et de ses homologues (voir Rosavear P. van Aken T. Baxter J., Fergusson-Miller S., Biochemistry 19, 4108, 1980 ; Shimamoto T, Saito S., Tsuchiya T., J. Biochem., 97, 1807, 1985 ; Schmidt R.R., Angews Chem. Intern. Ed. Engl., 25, 212, 1986 ; Straathof A.J.T., Romein F., van Rantwijk F., Kieboom A.P.G. et van Bekkum H., Starch, 39 (10), 362, 1987).

Ces techniques ne satisfont pas à la demande actuelle de tensio-actifs non ioniques, (notamment formant un système bicaténaire), ou anioniques et pouvant être préparés à des prix bas et d'une efficacité plus grande que ceux utilisés jusqu'ici.

Des tentatives récentes (D. Plusquellec et collaborateurs, Anal. Bioch. 179, 145 (1989) ; EP 334498 ; EP 427210 F. Bjorkling et collaborateurs, J. Chem. Soc., Chem. Commun, 934 (1989) ; K. Adelhorst et collaborateurs, Synthesis, 112-115 (1989) ; M.P. de Nijs et collaborateurs, Recl. Trav. Chim., Pays-Bas, 109, 429-433 (1990), permettent d'obtenir des agents tensio-actifs non ioniques de structure bien définie, et sans protection du substrat hydrophile. Toutefois, elles nécessitent une étape chimique de glycosilation et une étape enzymatique permettant le greffage d'un deuxième groupe alkyle sur la position alcool primaire du glucose. Les inconvénients majeurs d'une telle technique résident dans les limitations apportées par les lipases qui n'acceptent bien que certains substrats glucosidiques et dont les rendements de greffage sont très variables en fonction de la longueur de l'acide ou de l'ester gras mis en réaction. Si, dans quelques cas très favorables, les rendements peuvent atteindre 60 %, ils sont de façon plus générale inférieurs à 20 %. De plus, le coût du traitement enzymatique est loin d'être négligeable et la préparation d'un substrat alkylglycoside de bonne pureté n'est pas une opération facile.

Au EP-A-326 673, on décrit des saccharides solubles dans l'eau qui peuvent être utilisés comme détergents.

T. Böcker, J.Thiem Tenside Surf, Deterg. 1989, 26, pages 318 à 324 décrivent la synthèse et la caractérisation de nombreux glycosides d'alkyle non ioniques. A titre d'exemples, on peut citer des dérivés de monosaccharides tels que les α,β-glucopyranosides d'octyle, ou des dérivés de trisaccharides tels que les α,β-maltotriosides de dodécyle. L'influence de la nature de la tête polaire et de la queue hydrophobe sur les CMC a notamment été étudiée. Enfin, il est décrit un procédé permettant, en particulier, la transformation de certains glycosides d'alkyle non ioniques en tensioactifs anioniques par oxydation de l'hydroxyle primaire de ces composés. Le (n-dodécyl-α-D-glycopyranoside)-uronate de sodium a notamment été préparé en 4 étapes à partir du D-glucose.

Au Chem.Abs.1988, vol.109, abrégé 190679t, on décrit la synthèse de glucosides tensioactifs par la réduction asymétrique de phénylalkylcétones.

Au EP-A-326 673, on décrit le procédé d'oxydation de polysaccharides substitués, notamment des polyglucosides présentant jusqu'à 60 unités saccharidiques. Les oligosaccharides possèdent une chaîne alkyle ou acyle comportant de 8 à 22 atomes de carbone. Le procédé implique l'utilisation d'un catalyseur métallique du groupe VIII, notamment le platine. Trois exemples sont cités, deux concernent l'oxydation de glycosides d'alkyle et le dernier l'oxydation d'un polyglucoside d'alkyle présentant 2 à 4 unités glucoses. Les composés synthétisés présentent des propriétés tensioactives.

Par ailleurs, les composés décrits, notamment dans EP 427210 (Lion Corporation) et EP 334498 (Cerestar Holding), diffèrent de ceux de la présente invention puisque obtenus par esterification (enzymatique) par un acide gras de la position hydroxyle primaire d'alkyl glucosides. La généralisation, d'ailleurs hasardeuse (vu la spécificité de la catalyse enzymatique), de cette technique à l'ensemble des hexoses ne conduirait pas, même en série "galactose", aux composés qui font l'objet de l'invention.

En effet, les composés bicaténaires qu'obtiendraient ces auteurs en série "galactose" seraient de structure générale suivante : 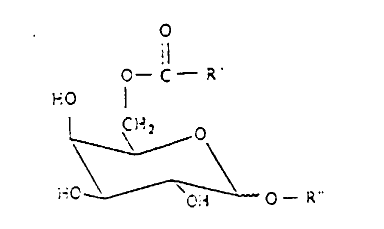 alors que les isomères "pyranose" de la présente invention sont de structure générale :

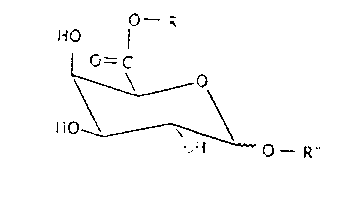

L'invention vise des dérivés de l'acide galacturonique qui peuvent être préparés sans phénomène d'oligomérisation, par voie purement chimique, sans protection préalable mais avec une régiosélectivité parfaite et des rendements élevés et qui s'avèrent doués de propriétés, notamment tensio-actives, supérieures à celles des meilleurs tensio-actifs utilisés jusqu'ici.

L'invention a pour objet des dérivés de l'acide galacturonique tels que définis aux revendications 1 à 4.

Parmi ces dérivés, on préfère pour leur bon pouvoir moussant ceux dans lesquels R1 est alkyle ayant de 8 à 14 atomes de carbone notamment les sels de l'acide dodécyle galactoside uronique dont le pouvoir moussant est supérieur aux produits classiques de référence. L'évaluation est réalisée selon la norme NFT 73404, qui consiste à faire s'écouler, selon un débit constant, 500 ml de solution de tensio-actif dans une éprouvette de 1000 ml graduée et thermostatée, contenant 50 ml de la même solution. La quantité de mousse, générée par l'écoulement, est estimée volumétriquement juste après la fin de l'écoulement (le paramètre mesuré est un volume initial de mousse).

La stabilité de la mousse est également prise en compte par mesure du volume durant 20 minutes.

Leur pouvoir moussant est sensiblement égal à celui des alkylpolyglucosides (APG) dont les coûts, en raison de leur procédé de fabrication et de purification (lié notamment à des problèmes de coloration et de stabilité en milieu basique du produit), dépassent le double de ceux des dérivés de l'invention.

Par ailleurs, ces composés présentent une très bonne stabilité dans le temps de la mousse, puisque le pourcentage de perte est inférieur à 5 % après 20 minutes.

Le test de pouvoir mouillant consiste à suivre durant 600 secondes, la quantité de solution de tensio-actif absorbée par un tissu de coton. La pièce de tissu vient effleurer la surface de la solution de tensio-actif et la traction générée par la montée capillaire de la solution est enregistrée en continu durant 600 secondes.

Le tissu utilisé est un coton écru découpé en pièces de (2 cm sur 2 cm, soit environ 0,12 g) et répondant aux spécifications de la norme NFT 73406. L'appareil utilisé est un tensiomètre KRUSS automatique, muni du logiciel d'adsorption K121. Les mesures sont effectuées à 25 °C.

Le détergent suivant l'invention imprègne ainsi mieux le linge à laver, au cours d'une lessive de plusieurs dizaines de minutes, que les détergents antérieurs.

La mesure des angles de mouillage est effectuée à l'aide du tensiomètre KRUSS muni du logiciel "K121 Contact Angle" contre une plaque de polyéthylène de 2 cm de longueur.

Ce logiciel permet l'enregistrement en continu du poids du polyéthylène durant sa pénétration de.5 mn dans la solution de tensio-actif. L'angle de contact à l'avancée est déterminé par extrapolation mathématique à l'origine.

Les résultats confirment l'intérêt des alkyl galactoside uronates dans le domaine de la détergence, notamment pour les chaînes alkyle ayant jusqu'à 14 atomes de carbone.

Les alkyl galactoside uronates préparés abaissent très efficacement la tension superficielle de l'eau. Cette propriété a été déterminée par une technique usuelle de tensiométrie, en utilisant un tensiomètre KRUSS de type K12, et selon la norme ISO 304. La mesure est effectuée à 25 °C. Le mobile de mesure est une lame de platine rectangulaire (25 mm x 5 mm).

L'appareillage entièrement automatique permet d'effectuer des mesures répétitives et de calculer la moyenne statistique de 10 valeurs.

Quand R1 a moins de 12 atomes de carbone, cet abaissement de tension superficielle est supérieur à celui observé avec les produits connus (Lauryl sulfate de sodium, lauryl éther sulfate de sodium à 2 mole d'oxyde d'éthylène, sodium dodécyl benzène sulfonate de sodium, alkyl glucoside, alkyl polyglucosides...) ; il est équivalent quand R1 a plus de 12 atomes de carbone. La mise en émulsion des salissures portées par du linge est ainsi rendue plus facile.

L'ensemble de ces propriétés (pouvoir moussant, mouillabilité, angle de mouillage, abaissement de la tension superficielle...) font que les alkyl galactoside uronates peuvent notamment être utilisés en détergence (domaine lessiviel notamment).

L'invention a également pour objet un procédé pour donner de la tensio-activité à une composition caractérisée en ce qu'il consiste à lui incorporer de 0,1 à 60 % en poids d'un dérive ou d'un mélange de dérivés suivant l'invention.

Une composition détergente en poudre suivant l'invention comprend de 0,1 à 60 %, et de préférence de 10 à 30 % en poids d'une base détergente et de 99,9 à 40 %, et de préférence de 90 à 70 % en poids d'adjuvants.

La base détergente peut être un dérivé ou un mélange de dérivés selon l'invention. Elle peut également être un mélange de un ou plusieurs dérivés selon l'invention avec un ou plusieurs tensio-actifs classiques dans le domaine ; ces tensio-actifs pouvant être anioniques, non ioniques, cationiques ou amphotères. La proportion de tensio-actifs selon l'invention représente de 1 à 100 %, en poids, et de préférence de 50 à 100 % de l'ensemble de la charge en tensio-actifs.

Les tensio-actifs anioniques de la composition, autres que ceux de l'invention, peuvent être des alkylbenzène sulfonates, des sulfates d'alcools gras, des éthers sulfates d'alcools gras, des α-oléfines sulfonates et parmi ceux relatifs à l'invention seront préférées ceux avec R1 étant alkyle ayant de 8 à 14 atomes de carbone. L'ensemble des tensio-actifs anioniques de la composition représentent de 30 % à 90 % en poids, et de préférence de 40 à 70 % en poids de l'ensemble de la charge en tensio-actifs. Les tensioactifs non ioniques de la composition, quand ils sont autres que ceux de l'invention, peuvent notamment être des éthers d'alkyl poly (éthylène glycol), des éthers de nonylphénylpoly (éthylène glycol), et, quand ils sont ceux de l'invention, sont des alkyl galactoside uronates d'alkyles de préférence de 6 à 12 atomes de carbone par chaîne alkyle.

Les adjuvants sont les "builders", les agents de blanchiement, et divers additifs tels les agents anti-redéposition, les agents anti-corrosion, des enzymes, des azurants optiques, des exhausteurs ou des régulateurs de mousse, des colorants, des parfums, des opacifiants...

Les builders peuvent être un phosphate, notamment un triphosphate, par exemple de métal alcalin, et en particulier de sodium, l'acide nitrilotriacétique ou ses sels de métaux alcalins, notamment de sodium, l'acide citrique ou ses sels, notamment de métaux alcalins, notamment de sodium, un acide glyconique, notamment l'acide gluconique ou l'acide galactonique, et ses sels, notamment de métal alcalin, et en particulier de sodium, un carbonate de métal alcalin, notamment de sodium, un acide polyacrylique ou ses sels, de métal alcalin notamment.

On peut utiliser ces builders en mélange selon différentes proportions. Le rapport de la charge de l'ensemble des builders sur celle de l'ensemble de la base tensio-active est compris-entre 1 et 4, et de préférence entre 2 et 3.

Les agents de blanchiement peuvent être un perborate, de métal alcalin notamment, et en particulier de sodium, un percarbonate, de métal alcalin notamment, et en particulier de sodium, et peuvent contenir ou non un activateur de blanchiement, notamment le tétraacétylglycolurile, le 1,5-diacétyl 2,4-dioxo-hexahydro- 1,3,5-triazine, le tétraacétyl éthylène diamine, la N,N-diacétyl N,N'-diméthylurée, les polyacétates de carbohydrates, en particulier d'hexoses ou de pentoses, et plus particulièrement de glucose, ou de saccharose, ou un stabilisateur de blanchiement, choisi parmi les stabilisateurs usuels, notamment le tétraacétate d'éthylène diamine (EDTA) ou des phosphonates. Ces agents de blanchiement représentent de 0,2 à 25 % en poids de la composition détergente en poudre.

Les agents anti-redéposition peuvent être des éthers de cellulose, notamment de carboxyméthyl cellulose, et leurs sels de métaux alcalins, notamment de sodium, ou des polymères synthétiques usuels dans ce type de formulation. Ils représentent de 0,5 à 3 %, et de préférence de 0,5 à 2 % en poids de la composition détergente. L'agent anti-corrosion peut être un silicate de métal alcalin, notamment de sodium, dont la proportion représente 0,5 à 25 % en poids de la composition détergente.

Les enzymes sont notamment des protéases ou des amylases. Les azurants optiques sont ceux utilisés classiquement dans le domaine, notamment l'acide stilbène disulfonique ou les dérivés du bis (styryl)biphényl. Les exhausteurs de mousse peuvent être alkyl éthanolamide, notamment le cocomonoéthanolamide. Les régulateurs de mousse peuvent notamment être des silicones, des savons ou des paraffines.

La composition détergente en poudre est utilisée à une concentration de 1 à 20 g/l, et de préférence entre 1 et 6 g/l. Le lavage est effectué dans une machine conventionnelle, entre 20 et 80 °C, et de préférence entre 20 et 60 °C, durant une période de 10 à 60 minutes.

Une composition détergente liquide suivant l'invention comprend de 0,1 à 60 %, et de préférence de 10 à 60 % en poids d'une base détergente de 99,9 à 40 % et de préférence de 90 à 70 % en poids d'adjuvants.

La base détergente peut être un dérivé ou un mélange de dérivés selon l'invention. Elle peut également être un mélange de un ou plusieurs dérivés selon l'invention avec un ou plusieurs tensio-actifs classiques dans le domaine ; ces tensio-actifs pouvant être anioniques non ioniques, cationiques ou amphotères. La proposition de tensio-actifs selon l'invention représente de 1 à 100 % en poids, et de préférence de 50 à 100 %, de l'ensemble de la charge en tensio-actifs. Les tensio-actifs anionique utilisés, quand ils sont autres que ceux de l'invention, peuvent notamment être un alkyl benzène sulfonate, un savon ou un éther sulfate d'alcool gras. Les tensio-actifs non ioniques utilisés, quand ils sont autres que ceux de l'invention peuvent notamment être des éthers d'alkyl poly (éthylène glycol), et quand ils sont ceux de l'invention, des alkyl galactoside uronates d'alkyles notamment avec alkyle ayant de 6 à 14 atomes de carbone. Lorsqu'un tensio-actif cationique est incorporé à la formulation il peut être un chlorure de dialkyldiméthyl ammonium.

Les ingrédients classiques complètent la formulation ; il s'agit notamment d'exhausteurs de mousse (0-2 %), d'enzymes, notamment de protéases (0 - 2 %) de builders, (0-30 % et de préférence de 10 à 30 %) notamment le citrate de sodium, le silicate de sodium, des zéolites ou de polycarboxylates, des stabilisants, notamment la tri et la monoétanolmine et des agents chelatants, des solvants de solubilisation (5-15 %), notamment l'éthanol ou le propylène glycol, (5-15 %), des azurants optiques, des argiles, des parfums, des colorants et de l'eau (30 - 60 %).

L'invention a également pour objet l'utilisation des dérivés selon l'invention pour obtenir une composition détergente pour le lavage de la vaisselle, et pour les usages ménagers. Cette composition comprend de 1 à 30 % en poids et de préférence de 5 à 25 %, de dérivés selon l'invention et 99,9 à 70 % en poids, et de préférence 95 à 75 % d'adjuvants. Ces adjuvants peuvent être d'autres tensio-actifs anioniques, notamment un sulfate d'alcool gras éthoxylé (0-20 % en poids), un épaississant, notamment le chlorure de sodium (0-5 %), un mono ou triéthanolamide d'acide gras éthoxylé ou non (0-15 %) un polyacrylate de sodium (0-5 % en poids) un complexant du calcium, notamment l'EDTA (0-5 %), un solvant notamment l'éthanol (0-5 %) un oxyde d'amine grasse (0-10 %) un parfum, un colorant, un conservateur... en quantités suffisantes.

Les compositions selon l'invention sont agréables au toucher et se rincent facilement.

La composition détergente liquide selon l'invention est utilisée en solution aqueuse à une concentration de 6 à 12 g/l, et à des températures de 40 à 70 °C.

L'invention a aussi pour objet des compositions cosmétologiques comprenant de 0,1 à 50 % et de préférence de 5 à 35 % en poids de substance active et de 99,9 à 50 % et de préférence de 95 à 65 % en poids d'excipients, caractérisé en ce que le détergent ou l'adoucissant est un dérivé de formule I selon l'invention. On préfèrera, pour leur faible concentration micellaire critique (0,3 à 1,2 g/l), l'octadécyl, l'hexadécyl et le tetradécyl galactoside uronates en raison de l'agressivité plus faible vis à vis des muqueuses et de la peau (Lang G. et Spengler I., Prépurits. IF SCC Congr., 1986, vol I p25). On préférera, pour leur bon pouvoir moussant, les décyl et dodécyl galactoside uronates de sodium notamment.

La composition cosmétologique peut être un savon liquide doux, contenant 5 à 30 % en poids, et de préférence 5 à 20 %, d'un dérivé selon l'invention et 95 à 70 % en poids, et de préférence 95 à 80 % en poids d'excipients. Ces excipients peuvent être un autre tensio-actif anionique notamment le cocoyl isethionate de sodium (0-10 % en poids), le lauryl sulfate de Na (0-10 %) le sel de sodium d'un alkyl peptide (0-15 % en poids), un tensio-actif amphotère, notamment un alkyl amidopropylbétaine tel le cocoamidopropyl bétaine (0-10 % en poids), une huile minérale lourde (0-20 % en poids), un dérivé cellulosique, notamment un carboxyméthyl cellulose (0-1 %), un solvant notamment un alcool tel l'éthanol ou le propylène glycol (0-5 % en poids), un complexant notamment l'EDTA (0-2 %), du chlorure de sodium (q.s), un alcool gras, notamment l'alcool cétylique (0-5 % en poids), des conservateurs, des parfums, des colorants (qs).

La composition cosmétologique peut être un shampooing, notamment un shampooing doux à usage fréquent. Il est composé de 5 à 35 % en poids d'une base détergente dont, de préférence, 10 à 75 % est constitué par un dérivé ou un mélange de dérivés selon l'invention, et de 95 à 65 % d'adjuvants.

Les autres tensio-actifs constituant la base détergente peuvent être un alkyl éther sulfate tel le lauryl éther sulfate de sodium ou de magnésium, un alkyl éther sulfate de sodium polyoxyéthylèné, tel le lauryl éther sulfate de sodium polyoxyéthyléné, une alkyl bétaine telle la cocoylbétaine, une alkyl amidopropyl bétaine telle la cocoyl amidopropyl bétaine, une alkyl diméthylamino acétique acide bétaine telle la lauryl diméthylamino acétique acide bétaine, une alkyl diméthylamino hydroxypropyl sulfobétaine un α-oléfine sulfonate de sodium, un alkyl polyéthylène glycol tel l'octadécyl PEG 15, une alkyl imidazolinium bétaine telle la cocoyl imidazolinium bétaine, un alkyl éther sulfosuccinate tel le lauryl éther sulfo succinate de disodium, un β alkyl amino propionate tel le sodium β-laurylaminopropionate, un alkyl diamino éthyl glycine tel le sodium lauryl diaminoéthylglycine et, lorsqu'ils sont des dérivés selon l'invention, ils sont de préférence des alkyls galactoside uronates avec alkyl ayant de 10 à 14 atomes de carbone.

Les adjuvants peuvent être des épaississants, des texturants, tels des diéthanolamide d'acides gras, notamment le cocoyl diéthanolamide tel un alkyl acrylate, notamment le laurylacrylate, le chlorure de sodium, un dialkylcarboxylate d'éthylène glycol tel le distéarate d'éthylène glycol, un N-oxyde d'amine grasse tel le N-cocoyl oxyde..., qui sont incorporés à hauteur de 0-10 % en poids dans la formulation. Les adjuvants peuvent être également des agents de conditionnement, des adoucissants tels des hydrolysats de protéines de blé, tels des éthers de cellulose contenant des ammoniums quaternaires (teneur en azote 1-3 %, PM = 50-150 000), un copolymère acrylamide / chlorure de diméthyl alkyl ammonium, représentant de 0,5 à 5 % en poids de la formulation, des complexants, notamment l'EDTA ou l'acide galactarique représentant 0,1 à 1 % en poids de la formulation, et finalement des parfums, des agents nacrants, des conservateurs, des acidifiants en quantité suffisante, et de l'eau purifiée.

La composition cosmétologique peut être un bain moussant contenant de 5 à 35 % d'une base détergente, elle même constituée de plus de 50 % de dérivés ou mélange de dérivés selon l'invention, et d'adjuvants. Les autres constituants de la base détergente sont les composés classiques dans le domaine et peuvent notamment être des alkyl amido bétaines, tel le cocoylamidopropyl bétaine, un alkyl carboxylate de sorbitan éthoxylé, tel le laurate de sorbitan éthoxylé.

Les adjuvants sont les mono ou tri éthanolamide d'acides gras (0-10 % en poids), un dialkylcarboxylate de propylène glycol éthoxylé (0-5 % en poids), un polyéthylène glycol, notamment le triéthylène glycol (0-5 % en poids), un alkyl acrylique, notamment un oleyl acrylique (0-5 % en poids), une huile végétale, notamment l'huile d'amandes douces (0-10 % en poids), du chlorure de sodium (qs), de l'EDTA (0-0,5 % en poids), un alcool gras notamment l'hexadécanol (0-2 % en poids), un conservateur, un parfum, un colorant en quantité suffisante et de l'eau.

La composition cosmétologique peut être un gel douche contenant 5 à 35 % d'une base détergente, elle-même constituée d'au moins 50 % de dérivés, ou mélange de dérivés suivant l'invention et d'adjuvants.

Les tensio-actifs pouvant compléter la base détergente lorsqu'ils ne sont pas ceux de l'invention, peuvent être un alkyl sulfosuccinate polyoxyéthyléné, tel le cocoyl sulfo succinate à 3 moles d'oxyde d'éthylène, un alkyl amido-N-glycinate tel le C12-C18 amido-N-glycinate, un alkyl sulfate d'ammonium, tel le lauryl sulfate d'ammonium.

Les adjuvants peuvent être un alkylcarboxylate de propylène glycol éthoxylé tel le dioléate de propylène glycol éthoxylé (0-5 % en poids), une alkyl amido bétaine tel un lauryl amidopropyl bétaine (0-5 % en poids), un agent nacrant (0-7 % en poids), un gel acrylique, (0-1 **%** en poids), du chlorure de sodium, un complexant, un conservateur, un parfum, en quantités suffisantes et de l'eau purifiée.

La composition cosmétologique peut également être une composition de type dentifrice, un bain pour l'hygiène buccale, un syndet....
La composition cosmétologique peut, notamment par incorporation d'alkyl galactosides uronate d'alkyles selon l'invention, d'octyl galactoside uronate d'octyl notamment qui forme une structure de type gel pour des concentrations en eau supérieures à 60 %, et de préférence supérieures à 90 %, être une crème de soins, pour le visage notamment.

Les dérivés selon l'invention sont également des cristaux liquides extrinsèques, notamment l'hexyl D-galactonide uronate d'hexyle et l'octyl D-galactoside uronate d'octyle lorsqu'ils sont à des concentrations comprises entre 5 et 60 % en poids, dans l'eau notamment.

On peut préparer ces acides alkyl galactosides uroniques par un procédé tel que défini à la revendication de procédé.

L'article galacturonique peut être préparé par hydrolyse de pectines (Anderson King J., J. Chem. Soc., 1961, page 5333).

A la différence de l'art antérieur, qui nécessite la réaction d'un acide ou d'un ester inférieur sur un sucre présentant un certain nombre d'hydroxyles sur chacun desquels l'acide ou l'ester réagit avec formation d'autant de chaînes latérales, le procédé suivant l'invention met en jeu la réaction d'un alcool sur un sucre possédant, outre une fonction alcool anomérique privilégiée, à savoir la fonction alcool portée par le carbone en alpha de l'oxygène endocyclique, une fonction acide carboxylique, en sorte que la réaction de l'alcool a lieu exclusivement sur ces deux positions, ce qui donne une régiosélectivité parfaite avec seulement deux chaînes latérales en des positions bien définies.

En outre, comme la réaction d'estérification (sur la fonction acide carboxylique) est plus rapide que celle de glycosidation (sur la fonction alcool anomère), le sucre de départ ou substrat, initialement peu soluble dans le milieu réactionnel constitué d'alcool additionné éventuellement d'un solvant, prend rapidement un caractère lipophile qui le rend plus soluble et favorise la réaction de glycosidation, qui peut dès lors se dérouler plus rapidement dans des conditions plus douces, ce qui se traduit par une moindre coloration du produit obtenu, tout en ayant un grand rendement.

### En pratique le procédé consiste :

Pour préparer les alkyl galactoside uronates d'alkyle:
- à mettre en contact un équivalent d'acide galacturonique;
- de 2 à 50 équivalents molaires, et de préférence de 2 à 10 équivalents molaires d'un alcool de formule R1 OH;
- de 10⁻³ à 1, et de préférence de 10⁻² à 10⁻¹ équivalent molaire d'un catalyseur acide, tel que l'acide chlorhydrique, l'acide sulfurique, un acide alkyl sulfurique tel l'acide décyl ou lauryl sulfurique, un acide sulfonique tel l'acide benzènesulfonique, l'acide paratoluène sulfonique, l'acide camphresulfonique, un acide alkylsulfonique tel l'acide méthylsulfonique, l'acide décylsulfonique, l'acide laurylsulfonique, l'acide sulfosuccinique ou un sulfosuccinate d'alkyl tel le sulfosuccinate de décyle ou sulfosuccinate de lauryle, les acides per halohydriques, tel que l'acide perchlorique, des métaux tels que le cuivre ou le fer, leurs oxydes ou leurs sels, comme leurs halogénures, des halogènes, comme l'iode, des pentahalogénures d'antimoine, notamment des pentachlorures ou pentafluorures, ou des sulfates de titane.

Cette catalyse acide peut également être effectuée par 0,05 à 6 équivalents pondéraux d'une résine sulfonique sous sa forme H⁺,ou d'une argile acide. Lorsque l'effet deshydratant de la résine est utilisé, on préfère pour cette catalyse hétérogène les résines à forte capacité de rétention d'eau.

On préfère l'acide sulfurique, un acide alkyl sulfurique, l'acide méthane sulfonique, un acide alkyl sulfonique, l'acide succinique ou un succinate d'alkyl, l'iode ou une résine sulfonique.
- De 2 à 20 équivalents en poids par rapport au substrat d'un solvant pouvant être un étheroxyde, tel le tétrahydrofuranne, le dioxanne, l'éther diméthylique de l'éthylèneglycol, l'éther diméthylique du diéthylèneglycol, un hydrocarbure halogéné tel le dichlorométhane, le chloroforme, le dichloroéthane, un ester tel que l'acétate d'éthyle, l'acétate de propyle ou l'acétate de butyle, un solvant nitré tel que le nitrométhane, le nitroéthane, le nitro-2- propane, un solvant de la famille des amides tel que le N-méthylformamide , le N,N-diméthylformamide, le N,N-diméthylacétamide, la N-méthyl-2 2-pyrrolidone, un nitrile tel que l'acétonitrile ou un alcane, de préférence l'hexane, l'heptane ou l'octane, ou un solvant aromatique tel que le toluène ou le xylène.

On peut également utiliser un mélange de deux ou plusieurs de ces solvants ou effectuer la réaction en l'absence totale de solvant.

Un agent de deshydratation classique, tel des tamis moléculaires ou des zéolites, qu'ils soient rajoutés directement dans le milieu réactionnel ou qu'on fasse circuler le filtrat de réaction à travers une colonne thermostatée emplie de cet agent de déshydratation, peut être utilisé.
- A effectuer la réaction à des températures comprises entre 25 et 140 °C et, de préférence, entre 50 °C et 90 °C et pendant une durée de 1 heure à 3 jours et, de préférence, de 3 heures à 24 heures ;
- A effectuer la réaction sous une pression comprise entre 0,1 et 760 mm Hg, et de préférence entre 0,1 mm et 300 mm Hg ;
- A filtrer le catalyseur acide, lors d'une catalyse hétérogène ou à neutraliser le catalyseur acide, puis à filtrer son sel lors d'une catalyse homogène. La neutralisation du milieu réactionnel est faite par exemple par un hydrogénocarbonate de métal alcalin, notamment l'hydrogénocarbonate de sodium.
- A évaporer le solvant et/ou l'excès d'alcool R₁OH pour récupérer le mélange des alkyles galactosides uronates d'alkyle de formule I.
- A chromatographier sur colonne de silice le mélange de ces alkyles galactosides uronates d'alkyle pour les séparer.

Les exemples suivants illustrent l'invention :

### 1) Exemple n° 1

Préparation de l'éthyl D-galactoside uronate d'éthyle :
(I, R1 = R2 = éthyle)

A 10 g (47,1 mmoles) d'acide D-galacturonique monohydraté mis en suspension dans 100 ml (79 g, 1714,7 mmoles) d'éthanol absolu, on ajoute 10 g de résine sulfonique Amberlyst 15 préalablement séchée. On porte le milieu réactionnel à 80 °C pendant 8 heures, puis on filtre. Après passage sur charbon actif, nouvelle filtration et concentration du filtrat sous vide, on obtient 10,6 g (90 % de rendement) d'une huile limpide légèrement jaunâtre correspondant à l'éthyl-D-galactoside uronate d'éthyle, présent majoritairement sous sa forme β-furanose, les autres formes étant, par ordre décroissant d'abondance, l'α-pyranose, l'α-furanose et le β-pyranose.

Une chromatographie flash du produit brut sur une colonne de silice (35 à 70 µm), en utilisant l'acétate d'éthyle comme phase mobile, permet d'isoler chacun des quatre isomères formés, dont les caractéristiques physico-chimiques sont résumées ci-après.

Rf en chromatographie sur couche mince :

Plaque de silice avec épaisseur de film de 200 µm et taille des particules de 5 à 10 µm, en utilisant l'acétate d'éthyle comme solvant de migration.

| | **Rf** |
|---|---|
| β-furanose | 0,47 |
| α-furanose | 0,41 |
| β-pyranose | 0,19 |
| α-pyranose | 0,15 |

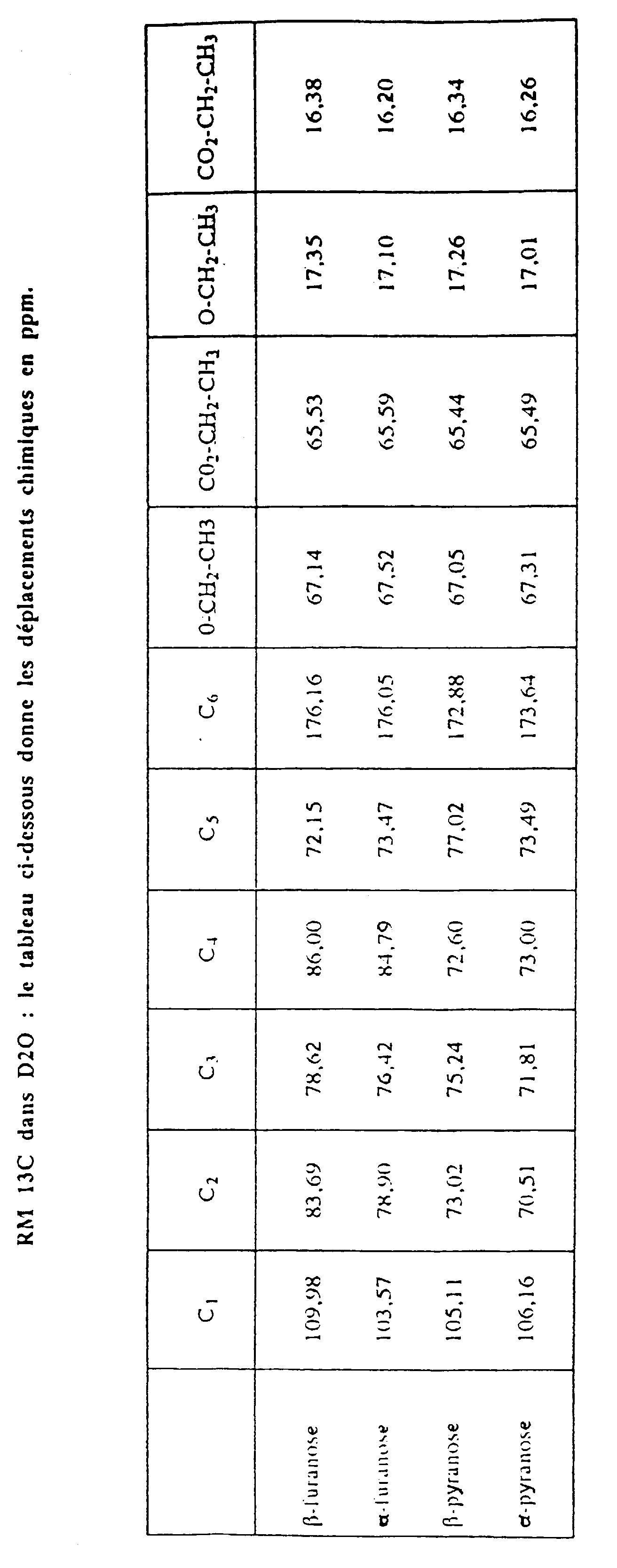

### 2) Exemple n° 2

Préparation du butyl-D-galactoside uronate de butyle
(I, R1 = R2 = butyle)

A 25 g (117,8 mmoles) d'acide D-galacturonique monohydraté mis en suspension dans 230 ml (186 g, 2510 mmoles) de n-butanol, on ajoute 0,4 ml (0,73 g ; 7,06 mmoles) d'acide sulfurique concentré, puis on porte le milieu réactionnel à 80 °C.

Après 3 heures de réaction, on refroidit, on neutralise la solution avec un excès d'hydrogénocarbonate de sodium, on filtre et on traite le filtrat au charbon actif. Après nouvelle filtration, on concentre le filtrat obtenu sous vide pour obtenir 33,5 g (93 % de rendement) d'une huile limpide jaune clair correspondant au butyl-Dgalactoside uronate de butyle dont les quatre formes isomères peuvent être séparées dans les conditions suivantes :
- Chromatographie "Flash" du produit brut obtenu sur colonne de silice de nature identique à celle décrite dans l'exemple n° 1 et en utilisant dans un premier temps le système éluant acétate d'éthyle/hexane - 70/30 en volume pour séparer les formes β-furanose et α-furanose, puis de l'acétate d'éthyle pour séparer les formes β-pyranose puis α-pyranose, dont les caractéristiques physico-chimiques obtenues par chromatographie sur couche mince effectuée dans les conditions décrites dans l'exemple n° 1 sont données ci-après.

| | **Rf** |
|---|---|
| β-furanose | 0,61 |
| α-furanose | 0,61 |
| β-pyranose | 0,45 |
| α-pyranose | 0,36 |

RMN¹³ C dans CDCl₃

| | C1 | C2 | C3 | C4 | C5 | C6 |
|---|---|---|---|---|---|---|
| β-furanose | 107,80 | 80,20 | 77,17 | 85,22 | 69,53 | 172,22 |
| α-furanose | 100,83 | 77,52 | 74,24 | 82,61 | 69,74 | 172,10 |
| β-pyranose | 107,89 | 70,59 | 73,09 | 69,95 | 73,97 | 168,26 |
| α-pyranose | 98,85 | 68,12 | 69,83 | 70,22 | 70,40 | 169,04 |

avec, pour les formes pyranoses, les déplacements ¹³C des groupes "butyle" suivants :

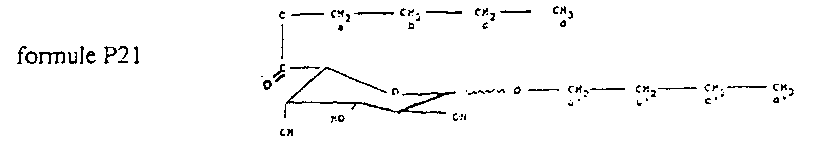

| ^{∂13}C des composés | a | a' | b | b' | c | c' | d | d' |
|---|---|---|---|---|---|---|---|---|
| β pyranose | 65,30 | 70,06 | 30,41 | 31,42 | 18,92 | 18,92 | 13,54 | 13,74 |
| α-pyranose | 65,04 | 68.38 | 31,30 | 30,3 | 19,01 | 18,77 | 13,55 | 13,39 |

### 3) Exemple n° 3

Préparation du n-hexyl-D-galactoside uronate de n-hexyle:
(I/R1 = R2 = n.-hexyle)

A 10 g (47,1 mmoles) d'acide D-galacturonique monohydraté, on ajoute 11,8 ml (9,6 g ; 94,2 mmoles) de n-hexanol et 2 ml (23,60 g ; 35,3 mmoles) d'acide sulfurique concentré. On pone puis on maintient le milieu réactionnel à 60 °C pendant 24 heures. On reprend le résidu avec 50 ml d'acétate d'éthyle, on ajoute un excès d'hydrogénocarbonate de sodium, on filtre et on traite le filtrat au charbon actif. Après nouvelle filtration, on concentre sous vide pour obtenir 13,7 g (80 % de rendement) du n-hexyl D-galactoside uronate de n-hexyle sous forme d'une huile limpide jaune. Une chromatographie flash faite dans les conditions de l'exemple n° 1 permet d'isoler ses 4 formes isomères. Elles ont les caractéristiques physico-chimiques suivantes.
. CCM faite dans les conditions de l'exemple n° 2 :

| COMPOSE | **Rf** |
|---|---|
| α-furanose | 0,68 |
| β-furanose | 0,63 |
| β-pyranose | 0,46 |
| α-pyranose | 0,36 |

RMN¹³ C dans CDCl₃ :

| | C1 | C2 | C3 | C4 | C5 | C6 |
|---|---|---|---|---|---|---|
| β-furanose | 107,90 | 80,17 | 77,90 | 85,35 | 69,58 | 171,97 |
| α-furanose | 100,89 | 77,59 | 74,25 | 82,75 | 69,77 | 170,90 |
| β-pyranose | 102,87 | 77,66 | 73,06 | 69,89 | 73,91 | 168,22 |
| α-pyranose | 98,85 | 68,36 | 70,08 | 70,27 | 70,41 | 169,03 |

### 4) Exemple n°4

Préparation de n-octyl D-galactoside uronate de n-octyle
(R1 : R2 : n-octyle)

A 50 g (0,235 mole) d'acide D-galacturonique monohydraté, on ajoute 600 ml (494 g ; 3,80 moles) de n-octyle et 30 g de résine S 100 de chez Bayer (type gel). On porte à 80 °C, et après 30 heures de réaction, on filtre la résine, on traite le filtrat au charbon actif, et on concentre sous le vide de la pompe à palettes (température de 73 °C et vide de 145 mPa) pour obtenir 90,7 g (92 % de rendement du n-octyl D-galactoside uronate de n-octyle sous forme d'une huile orangée. Une chromatographie flash effectuée dans les mêmes conditions que dans l'exemple 1 permet d'isoler ses quatre formes isomères, dont les caractéristiques physico-chimiques sont données ci-dessous.
. CCM effectuée dans les conditions de l'exemple n° 2.

| COMPOSE | **Rf** |
|---|---|
| α-furanose | 0,72 |
| β-furanose | 0,66 |
| β-pyranose | 0,52 |
| α-pyranose | 0,42 |

RMN¹³ C dans CDCl₃ :

| | C1 | C2 | C3 | C4 | C5 | C6 |
|---|---|---|---|---|---|---|
| β-furanose | 107,95 | 80,30 | 77,16 | 85,31 | 69,58 | 171,97 |
| α-furanose | 100,93 | 77,79 | 74,58 | 82,83 | 69,82 | 167,51 |
| β-pyranose | 102,90 | 70,68 | 73,09 | 69,91 | 74,01 | 167,60 |
| α-pyranose | 98,96 | 68,18 | 69,87 | 70,30 | 70,42 | 167,25 |

### 5) Exemple n° 5

Préparation du n-décyl-D-galactoside de n-décyle (I/R1 = R2 = n-décyle).

A 200 g (0,940 mole) d'acide D-galacturonique monohydraté, on ajoute 1000 ml (829 g ; 5,237 moles) de n-décyle et 5 g (26,8 mmoles) d'acide paratoluène sulfonique monohydraté. On porte à 60 °C, sous un vide de 260 mmg de mercure pendant 24 heures. Après distillation de l'excès de n-décanol, on récupère 473 g une huile limpide jaunâtre contenant environ 50 g de n-décanol résiduel. Rendement en n-décyle galactoside uronate de n-décyle: 473 g (95 %).

Une chromatographie sur colonne de gel de silice (35 à 70 µm) en utilisant comme éluant un système éther de pétrole/ éther éthylique (1/1, V/V), puis de l'éther éthylique, et enfin un système éther éthylique/acétate d'éthyle (6/4 - V/V), permet de séparer les 4 isomères formés. Les isomères α pyranose, β pyranose et β furanose cristallisent après évaporation du solvant d'élution. Ils sont ensuite recristallisés.

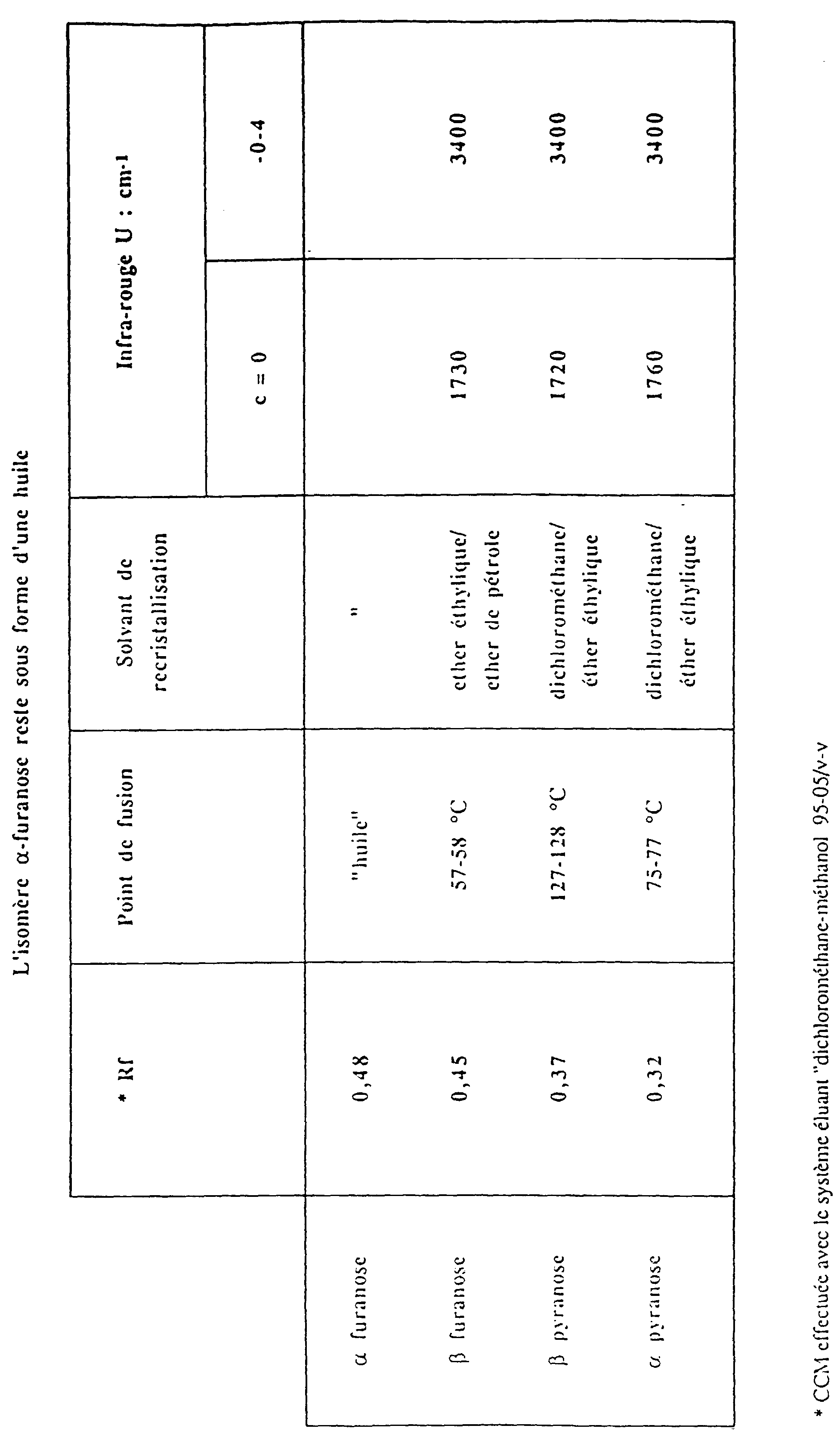

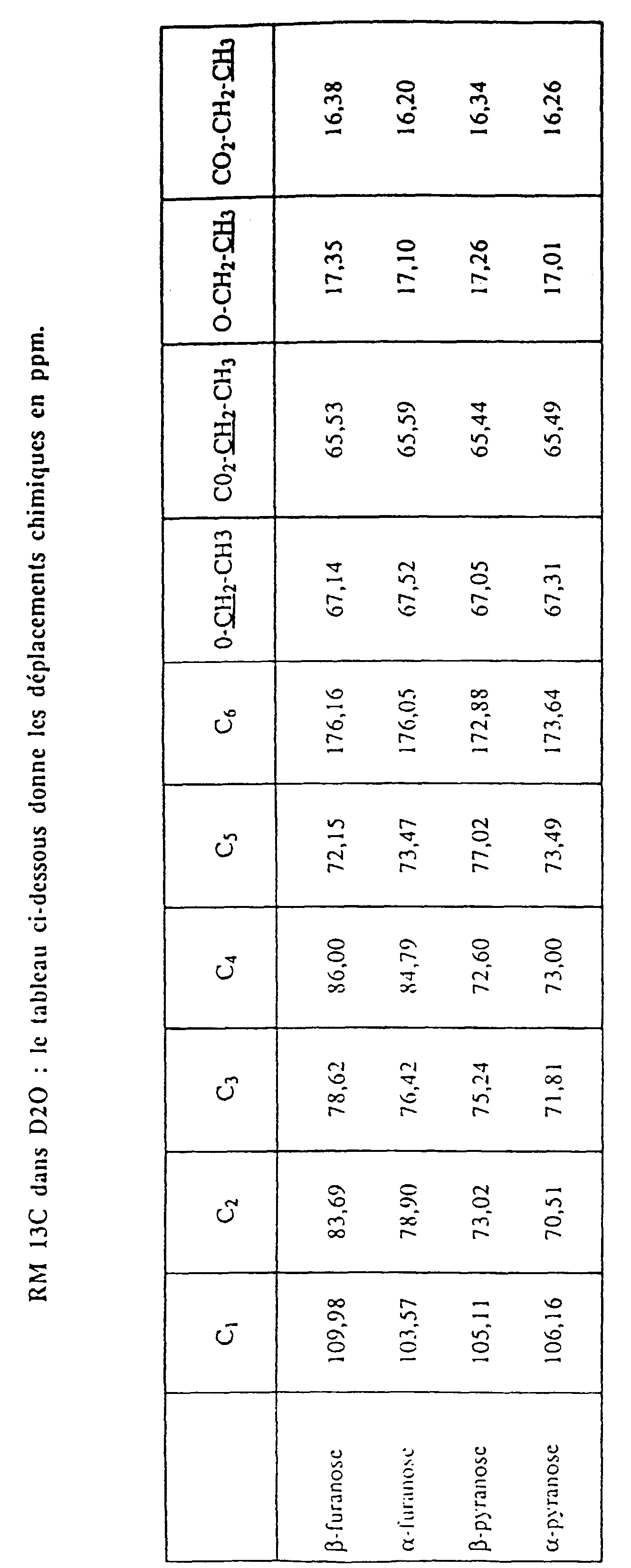

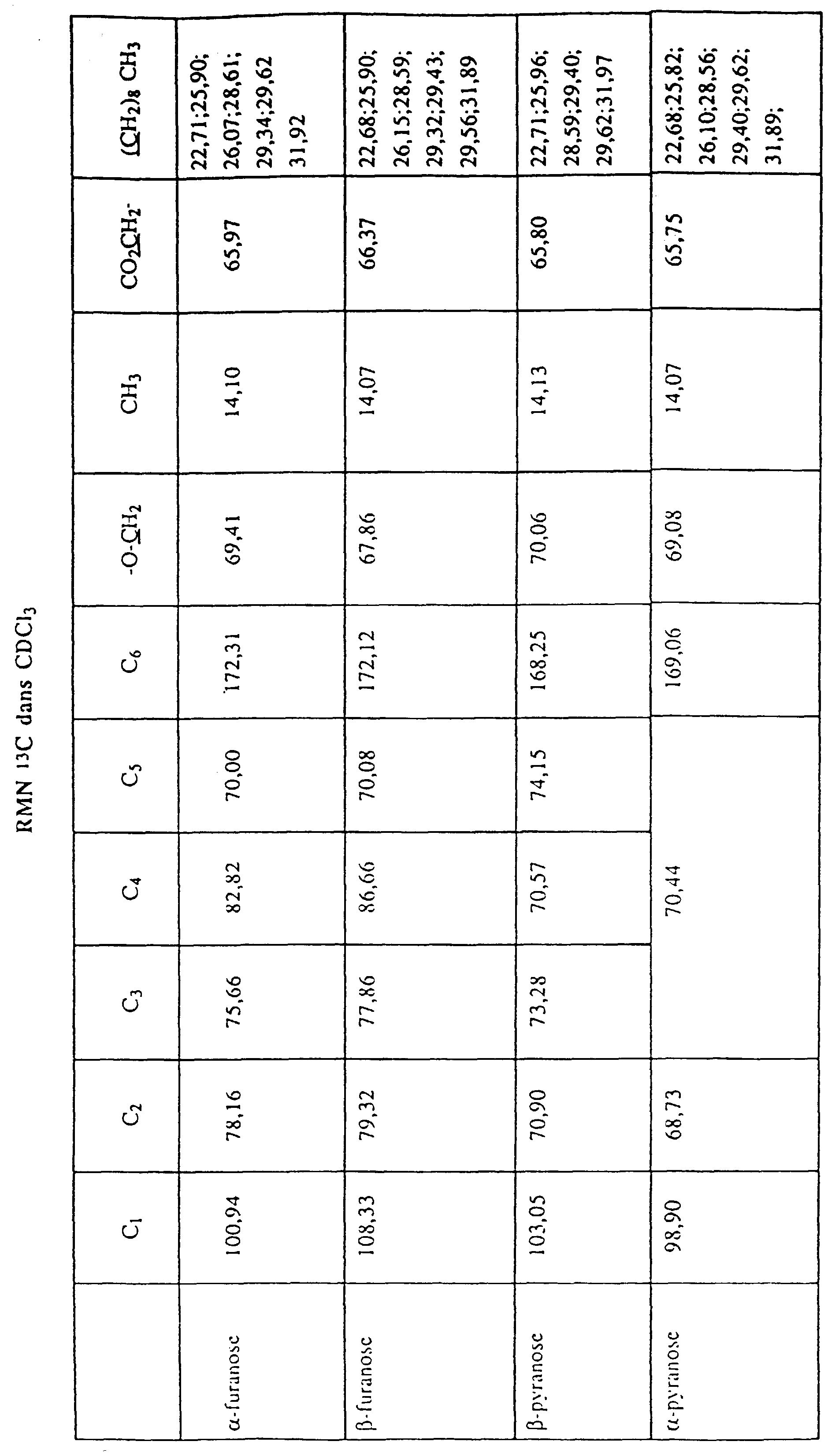

### 6) Exemple n°6

***Préparation du n-dodécyl D-galactoside uronate de n-dodécyle***
***(I/R1 = R2 = dodécyle)***

### Méthode A :

A 200 g (0,94 mole) d'acide D-galacturonique monohydraté, on ajoute 800 ml de diglyme, 702 g (3,77 moles) de n-dodécanol, et 7,36 g (0,08 mole) d'acide sulfurique concentré. On chauffe à 60 °C sous un vide de 18 mm de mercure pendant 24 heures. On amène à pH = 7 par de l'hydrogénocarbonate de sodium. On filtre les sels formés et on concentre le filtrat. Après chromatographie sur gel de silice, on récupère avec un rendement global de 70 % des isomères majoritaires n-dodécyl D-galactoside uronate de n-dodécyle (même système éluant que dans l'exemple n° 6).

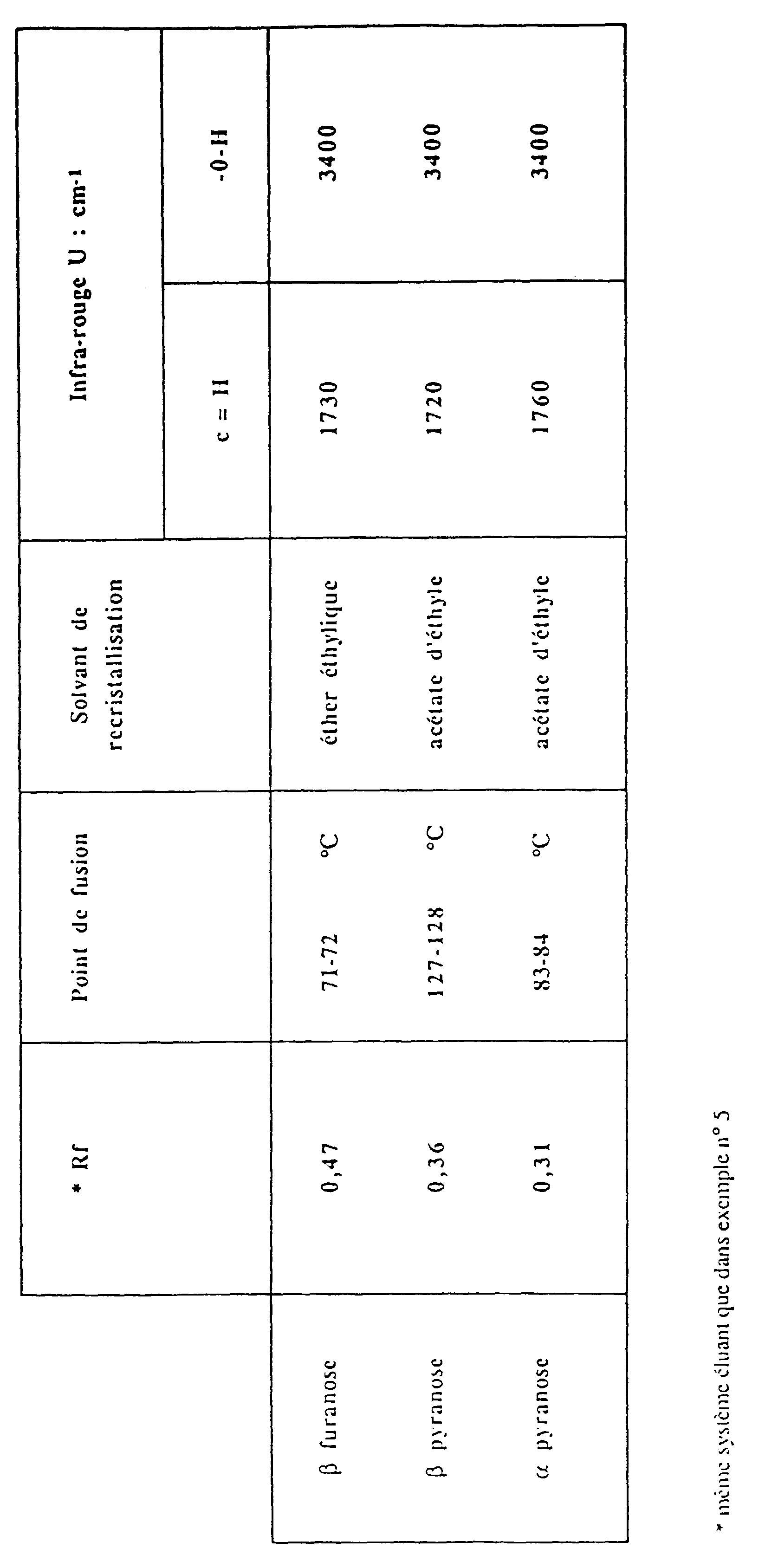

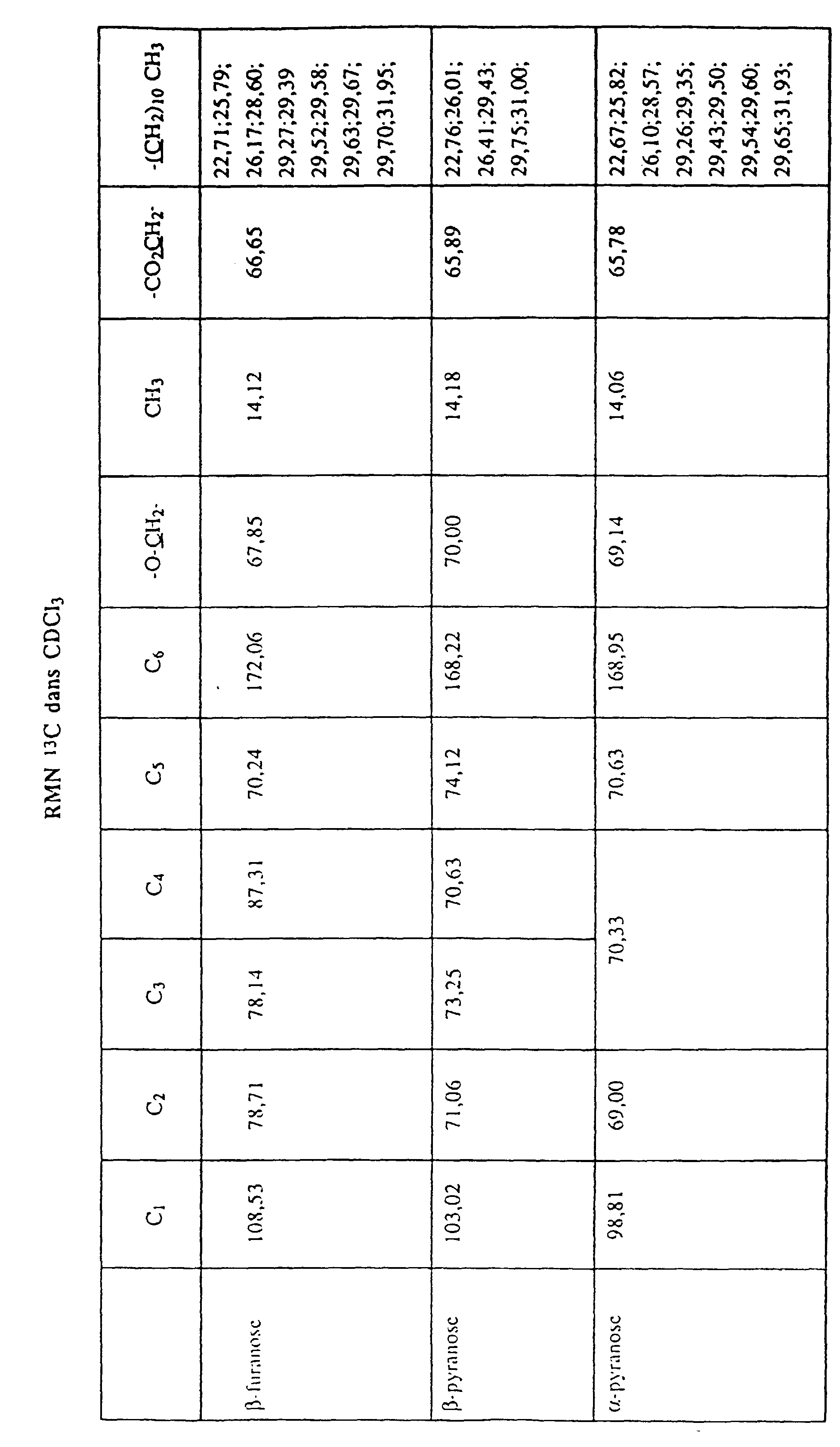

### Méthode B :

Effectuée avec les mêmes charges d'acide D-galacturonique et d'alcool gras que dans la méthode A, mais en utilisant comme catalyseur acide 4 g d'acide sulfosuccinique, et en conduisant la réaction à 70 °C sous un vide de 60 mm de mercure. Après 12 heures de réaction, le milieu réactionnel est chromatographié sur gel de silice (mêmes systèmes éluant que dans l'exemple n° 6). On récupère avec un rendement global de 80 %, les isomères majoritaires du dodécyl D-galactoside uronate de n-dodécyle décrits précédemment.

### 7) Exemple n°7

***Préparation du n-tetradécyl galactoside uronate de n-tetradécyle***
***(I/R1 = R2 = n-tetradécyle)***

Préparé selon l'exemple 7, méthode B, avec un rendement global 85 %. Le catalyseur acide utilisé est l'acide paratoluène sulfonique monohydraté (5 g).
(voir tableaux pages 33-34)

### 8) Exemple n°8

***Préparation du n-hexadéyl galactoside uronate de n-hexadécyle***
***(I/R1 = R2 = n-hexadécyle)***

Préparé selon l'exemple 7, méthode B, avec un rendement global de 90 % mais le catalyseur acide utilisé est l'acide paratoluène sulfonique (6 g), et le système d'élution pour la purification chromatographique est le suivant : dichlorométhane jusqu'à la sortie du n-hexadécanol en excès, puis le système dichlorométhane/méthanol (98/02-v/v). Les 4 isomères isolés cristallisent après évaporation des solvants d'élution et sont ensuite recristallisés. (Voir tableaux pages 35-36)

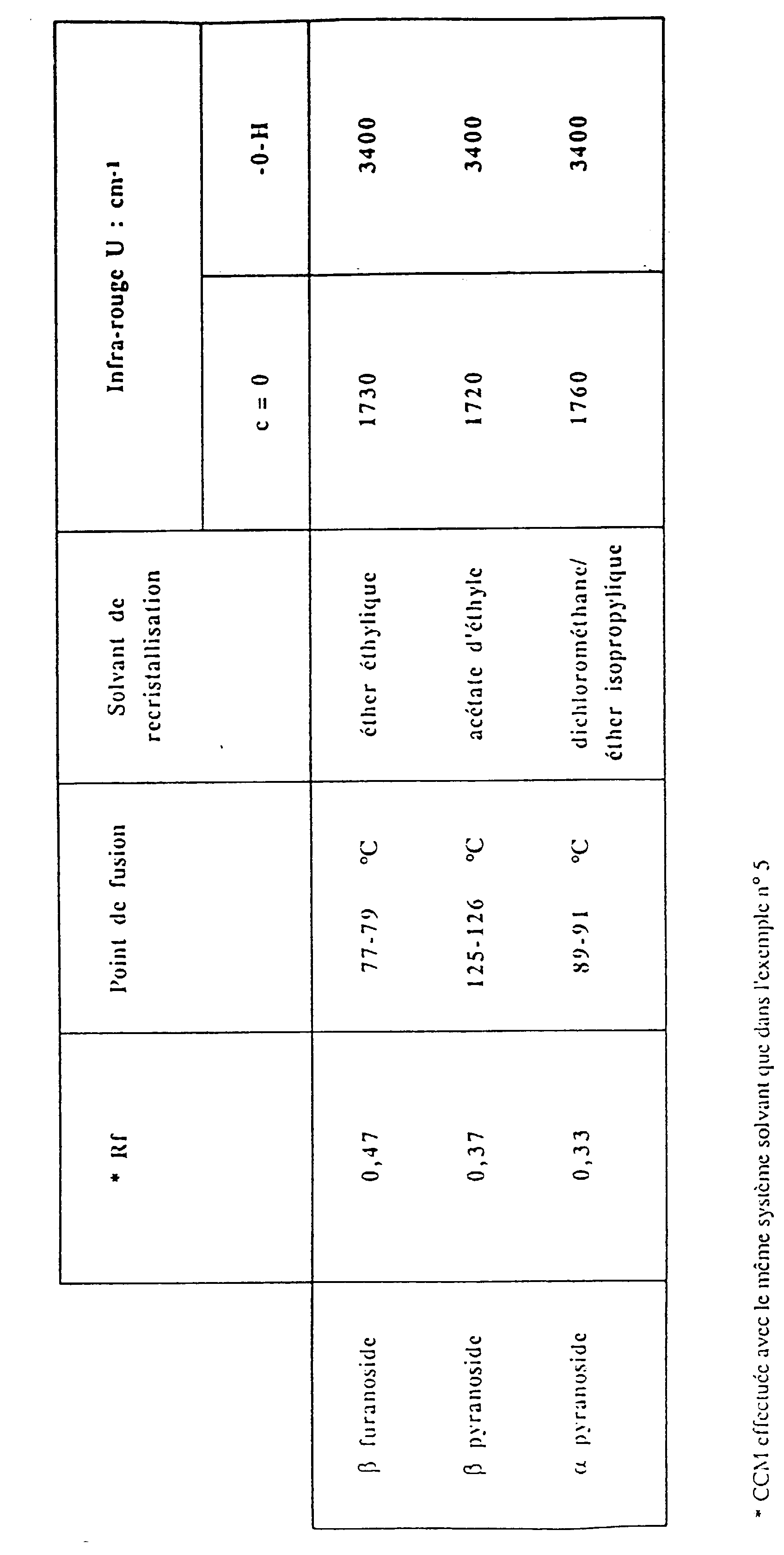

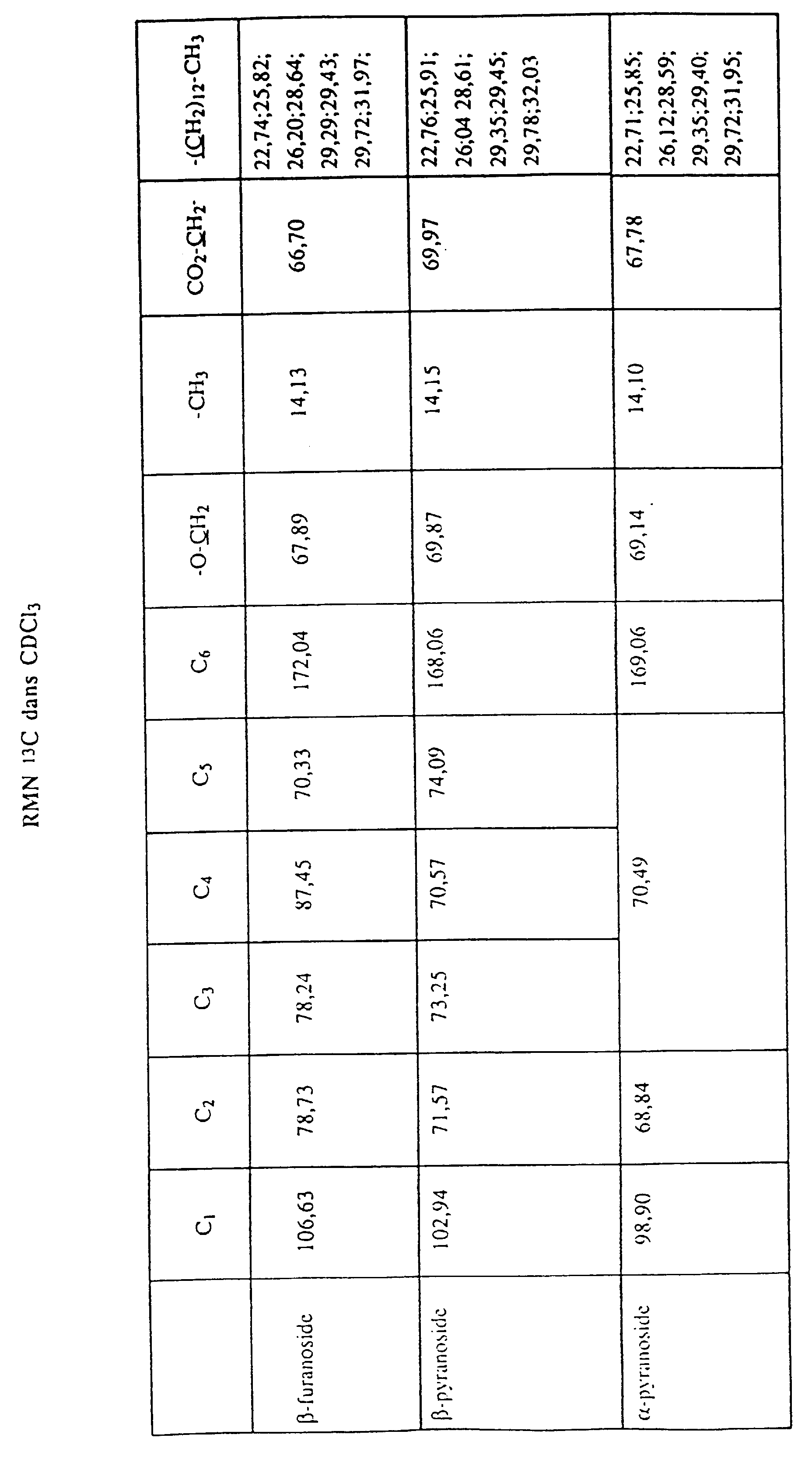

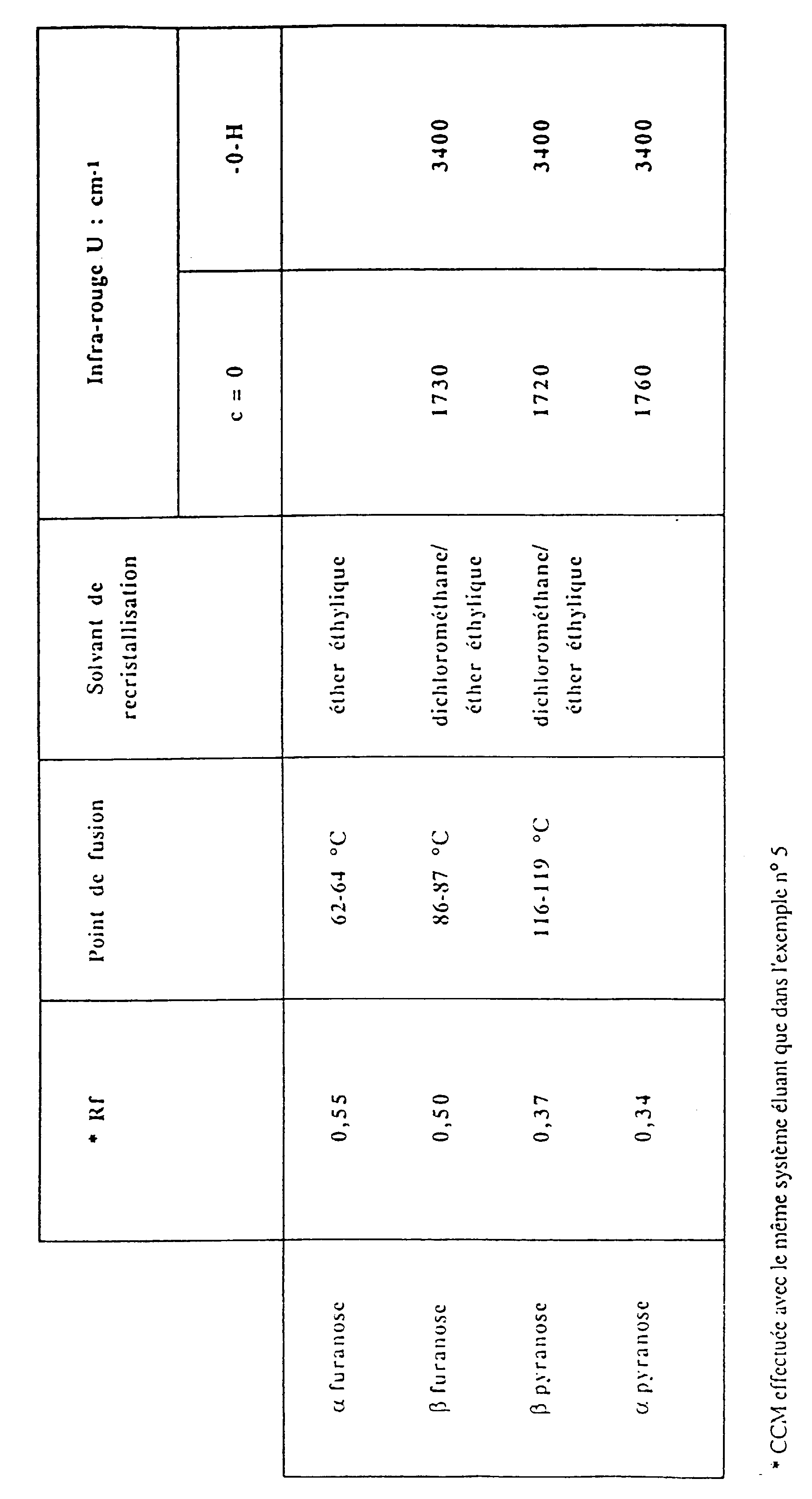

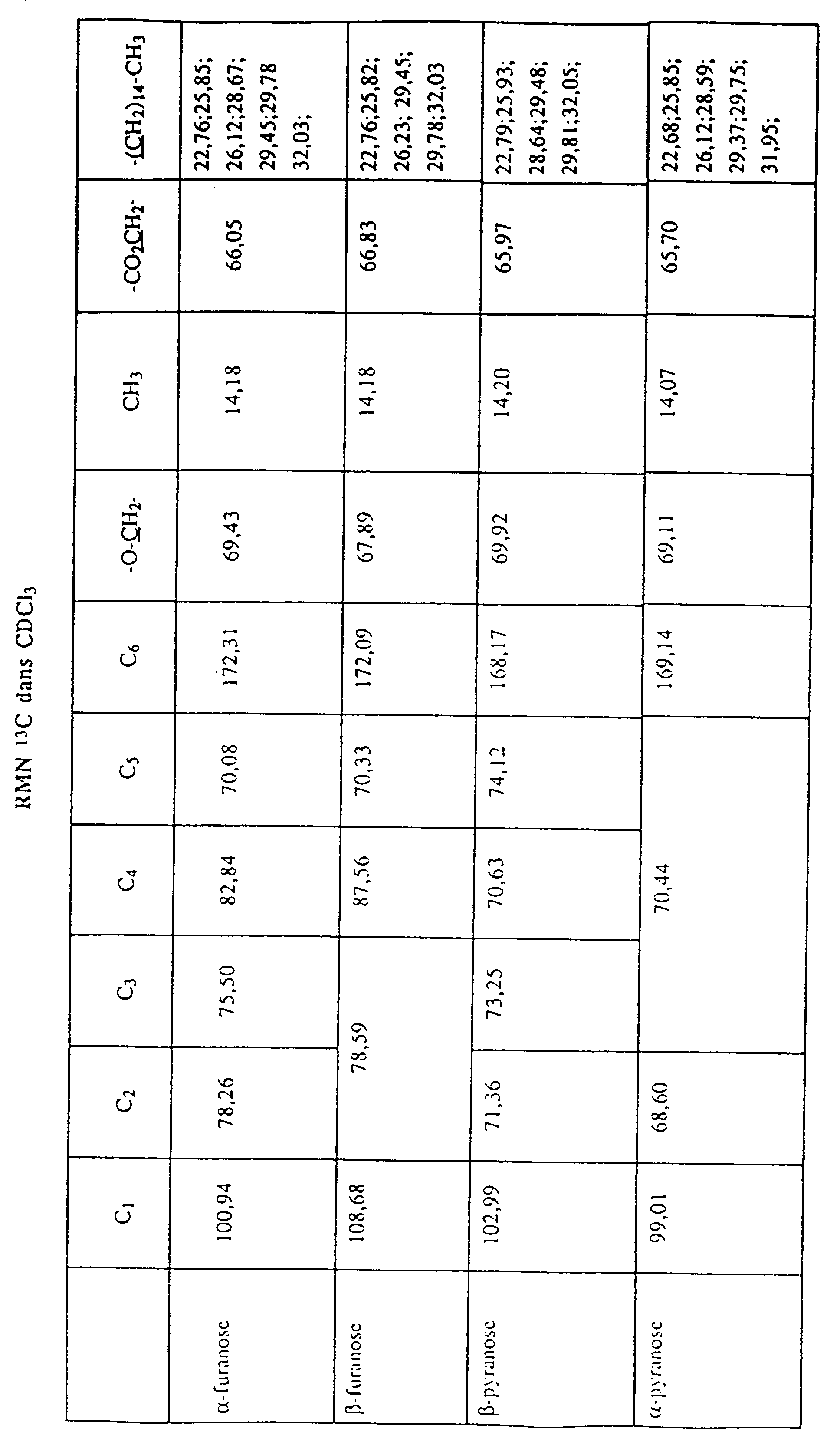

### 1) Exemple n° 9

**Préparation du n-octadécyl D-galactoside uronate de n-octadécyle**
**(I/R1 = R2 =n-octadécyle)**

### Méthode A :

A 200 g (0,94 mole) d'acide D-galacturonique monohydraté, on ajoute 508,5 g (1,88 mole) de n-octadécanol, 800 ml de diglyme et 10,2 g (5,53 ml ; 0,10 mole) d'acide sulfurique concentré. On porte à 60 °C sous un vide de 18 mm de Hg, pendant 10 heures. On ajoute alors de l'hydrogénocarbonate de sodium dans le milieu réactionnel jusqu'à l'obtention d'un pH de 7. On laisse refroidir ; le milieu réactionnel qui se solidifie est additionné de 500 ml de tétrahydrofuranne, agité, filtré sur fritté n°3. Du filtrat concentré précipite le n-octadécyl-D-galactoside uronate de n-octadécyle avec un rendement global de 94 %. Une chromatographie analogue à celle effectuée dans l'exemple 9 permet l'isolement des trois isomères majoritaires qui précipite lors de l'évaporation des solvants d'élution et qui sont ensuite recristallisés.

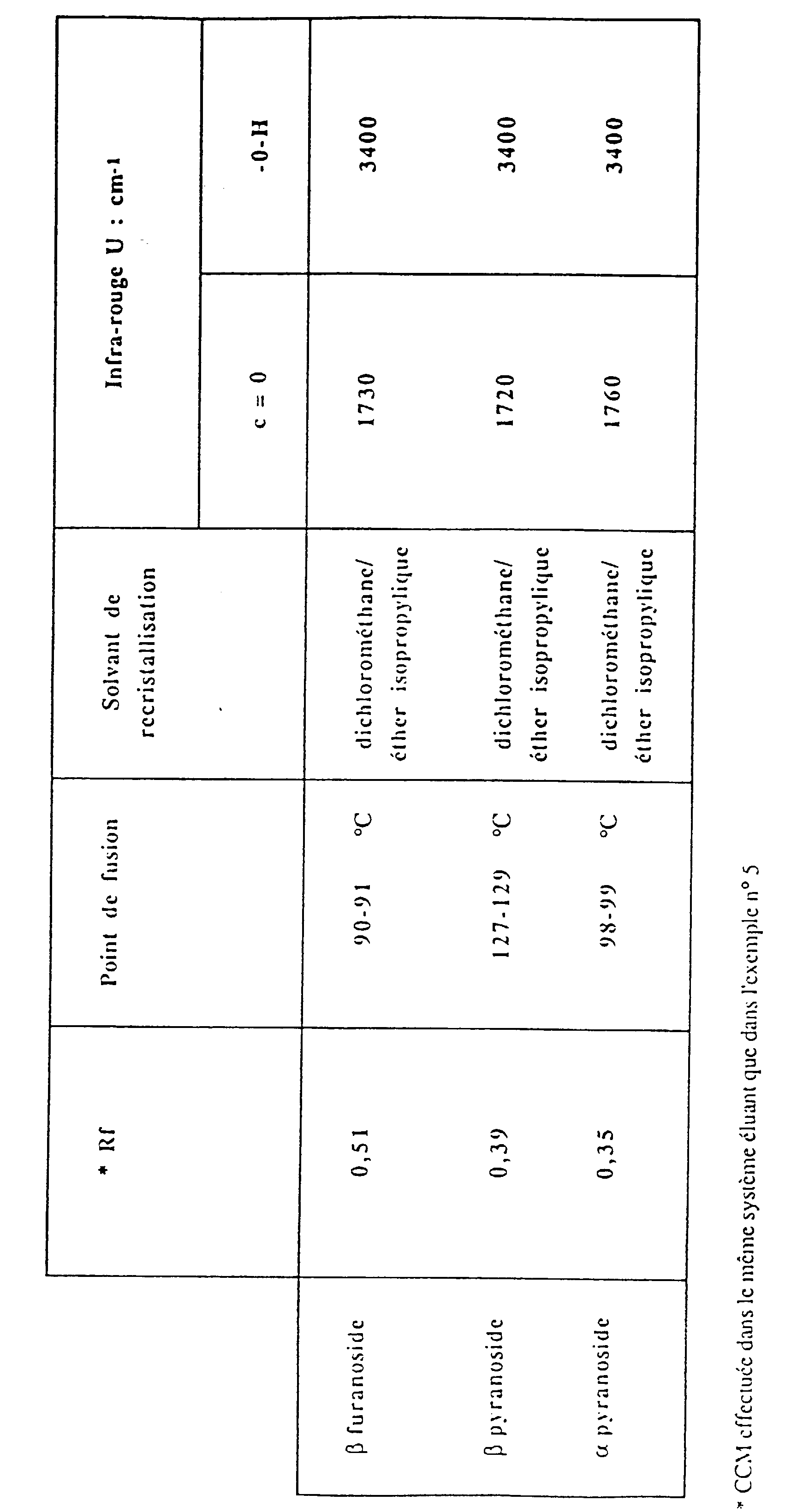

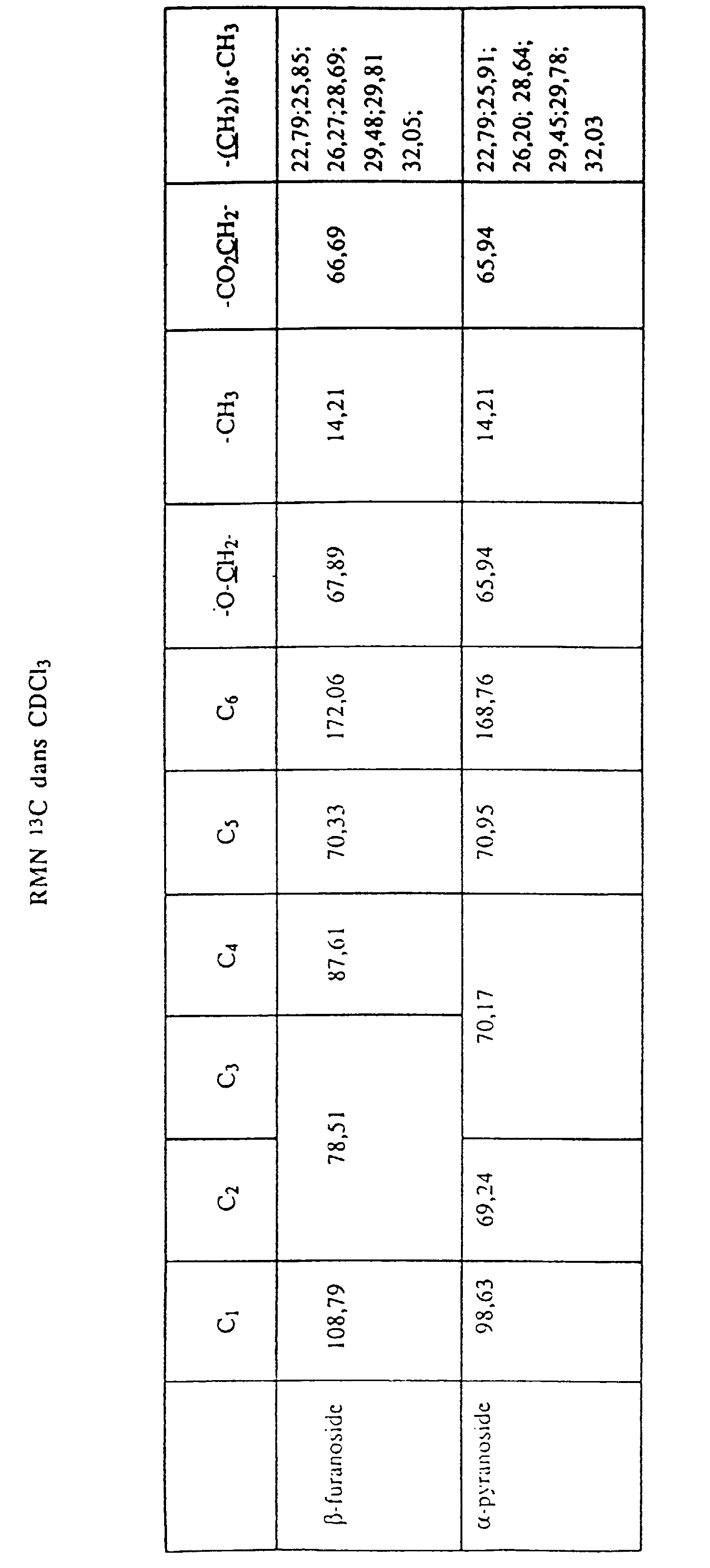

### EXEMPLES N° 33 A 35 :

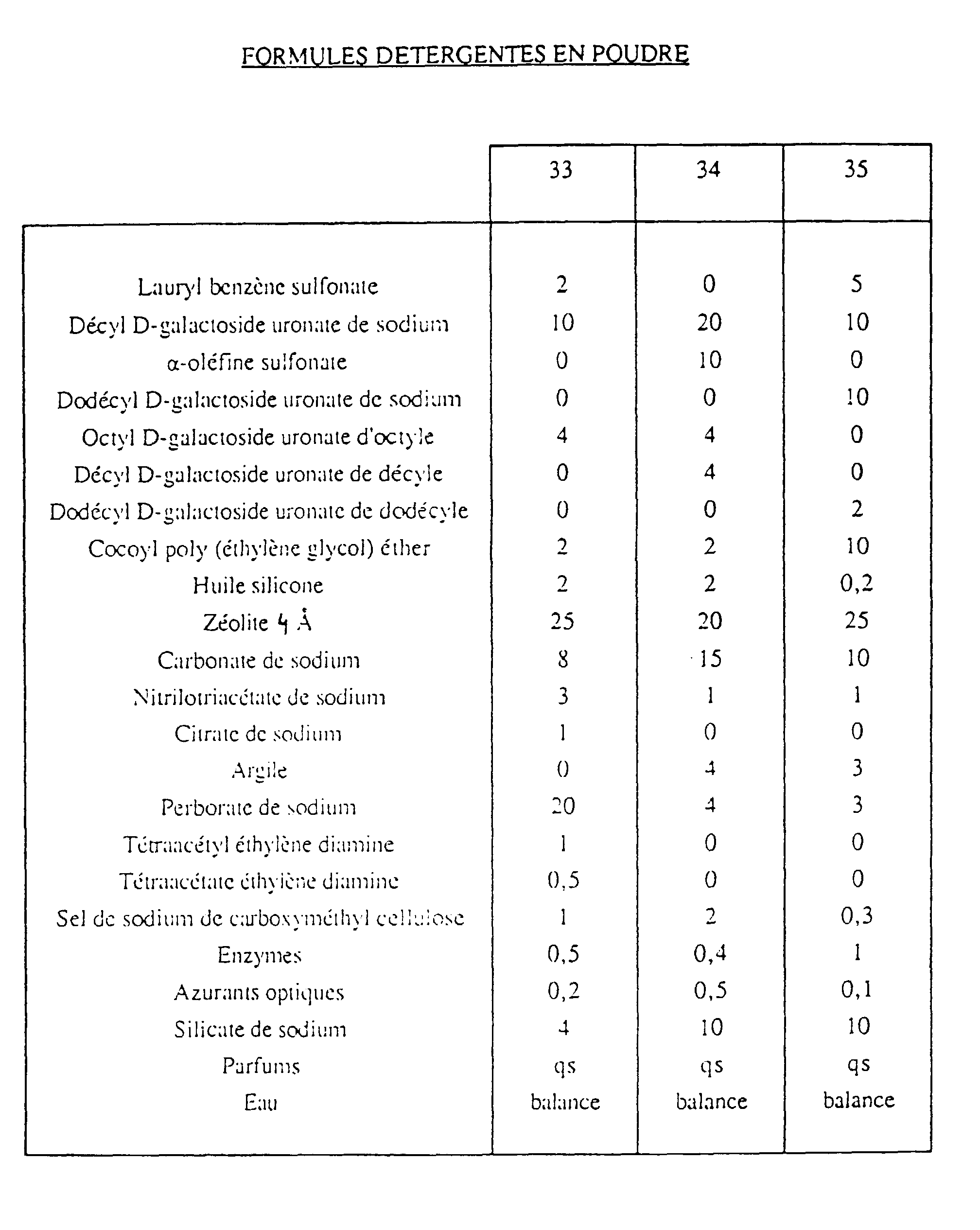

### EXEMPLES N° 36 ET 37 :

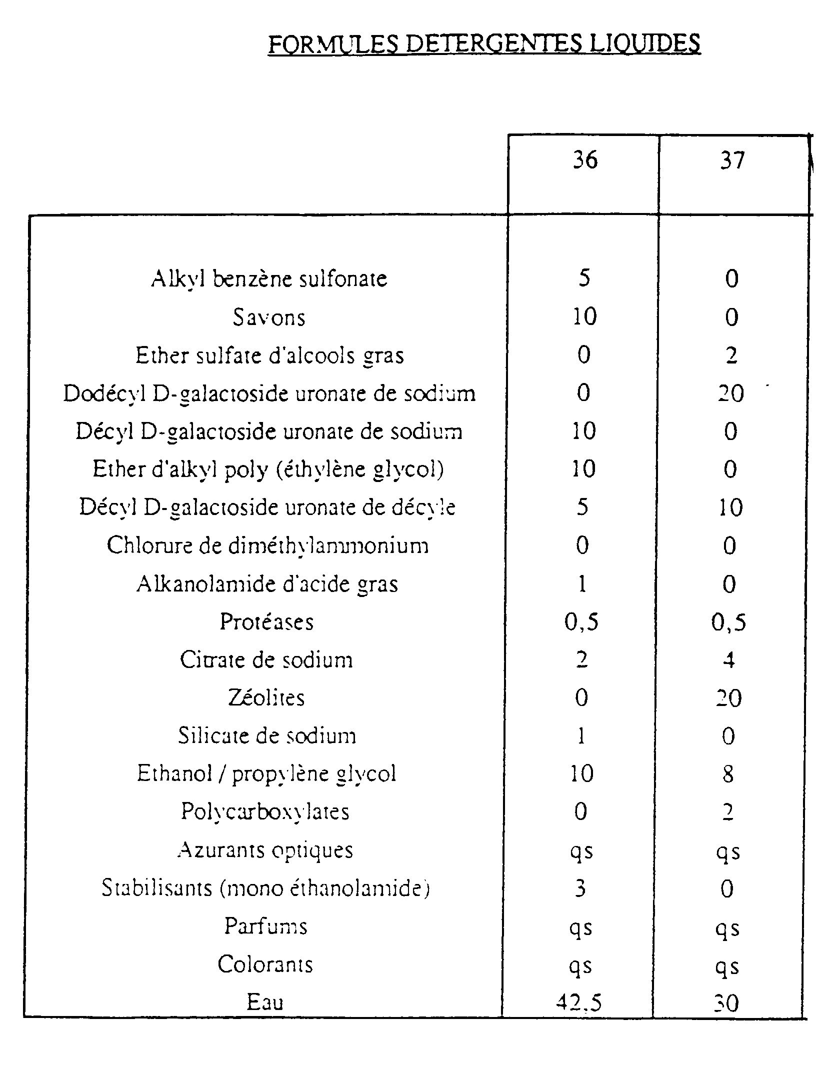

### EXEMPLE N° 38 :

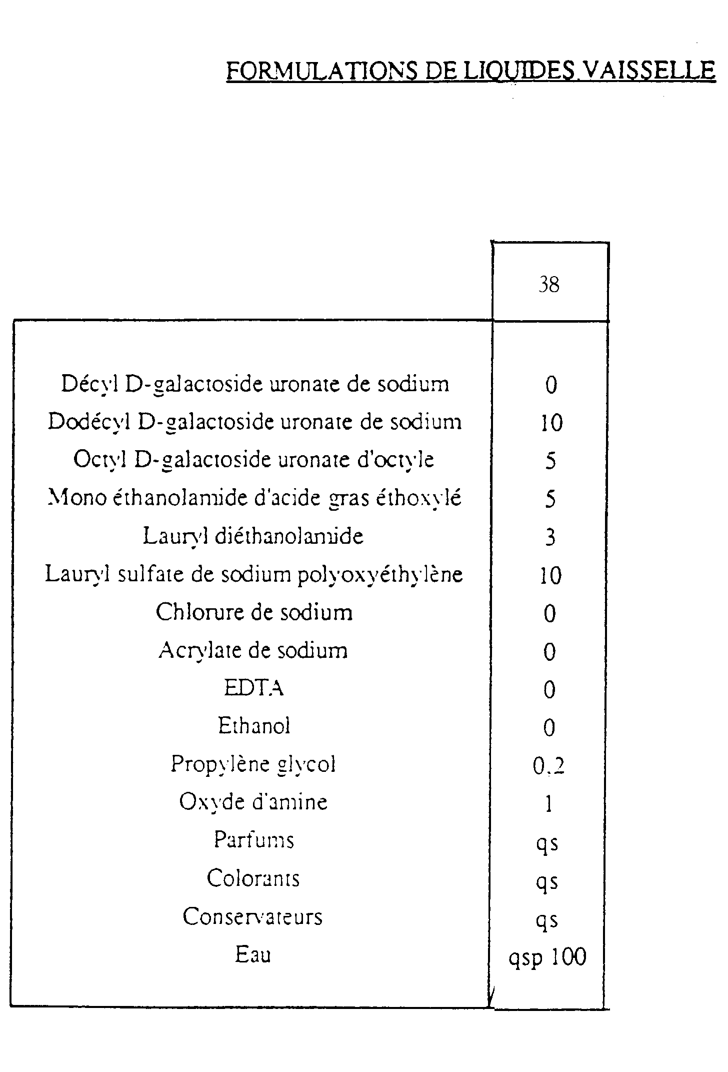

### EXEMPLES N° 39 ET 40 :

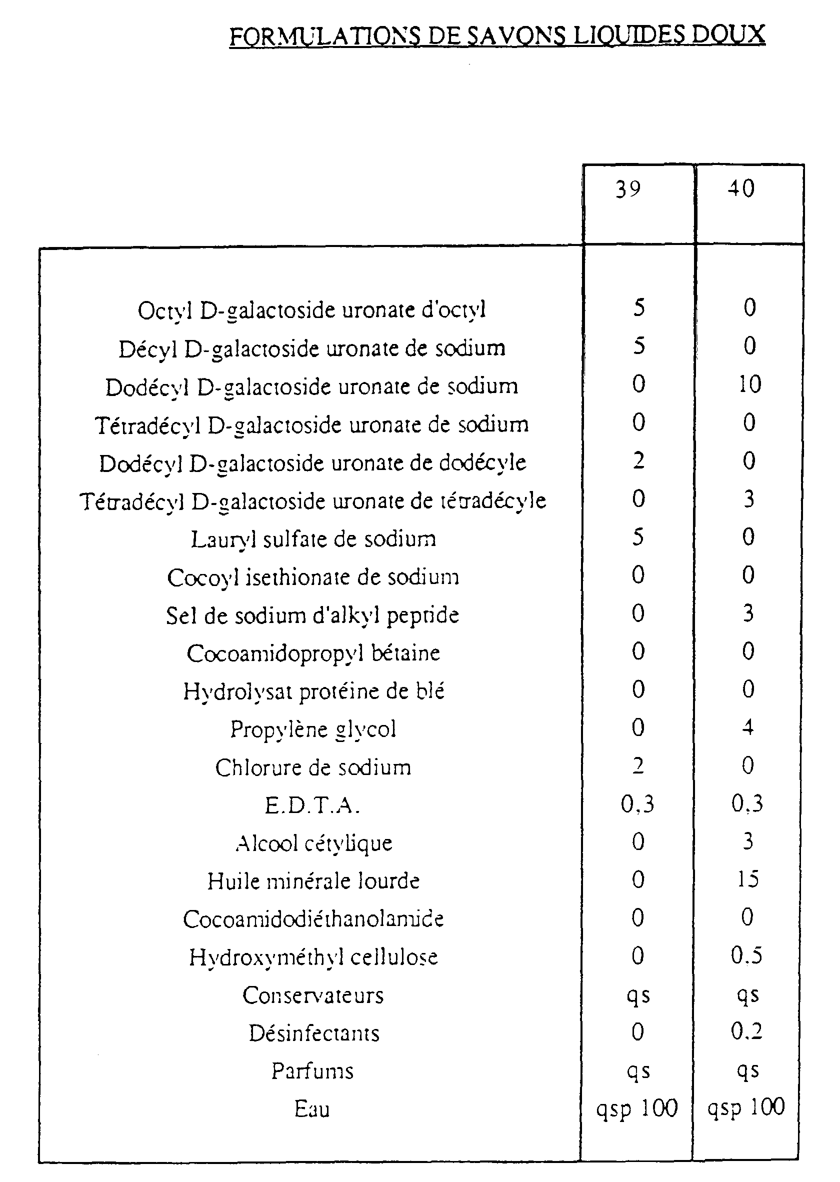

## Revendications (Revendications pour l'(les) Etat(s) contractant(s) suivant(s): AT, BE, CH, DE, DK, FR, GB, GR, IT, LI, LU, MC, NL, PT, SE)

1. Dérivé de l'acide galacturonique de formule: 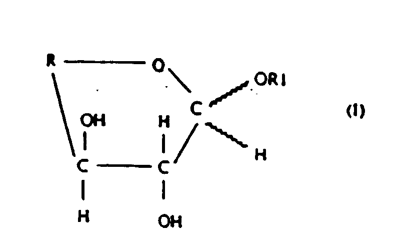 R1 étant alkyle linéaire ou ramifié ayant de 2 à 22 atomes de carbone,
R étant 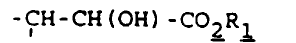 ou 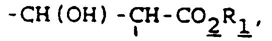 dont le carbone portant le groupe hydroxyle n'est pas relié à l'atome d'oxygène endocylique.

2. Dérivé suivant la revendication 1, caractérisé en ce que R1 est alkyle ayant de 8 à 14 atomes de carbone.

3. Dérivé suivant la revendication 2, caractérisé en ce que R1 est décyle.

4. Dérivé suivant la revendication 1, caractérisé en ce que R1 est alkyle ayant de 14 à 22 atomes de carbone.

5. Procédé de préparation de dérivés de l'acide galacturonique de formule (I), caractérisé en ce qu'il consiste, à faire réagir un alcool de formule R1OH sur l'acide galacturonique de formule: 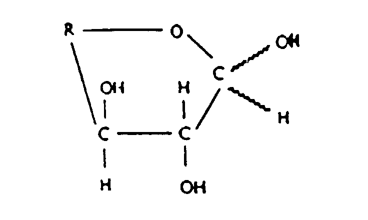 pour obtenir un mélange de formule 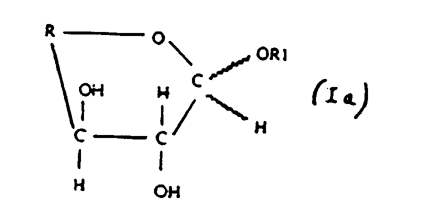 et à séparer chaque alkylgalactoside uronate d'alkyle du mélange.

6. L'utilisation d'un dérivé tel que défini aux revendications 1 à 4 comme agent tensio-actif.

7. L'utilisation d'un dérivé tel que défini aux revendications 1 à 4 comme détergent.

8. L'utilisation selon la revendication 7, caractérisé en ce que R1 est alkyle ayant de 8 à 14 atomes de carbone.

9. L'utilisation d'un dérivé tel que défini aux revendications 1 à 4 en cosmétologie.

## Revendications (Revendications pour l'(les) Etat(s) contractant(s) suivant(s): ES)

1. Procédé de préparation de dérivés de l'acide galacturonique de formule: 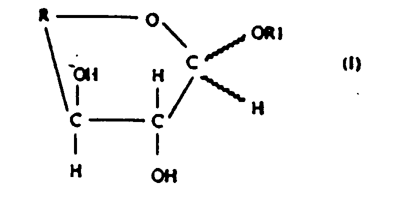 R1 étant alkyle linéaire ou ramifié ayant de 2 à 22 atomes de carbone,
R étant 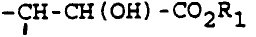 ou 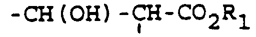 dont le carbone portant le groupe hydroxyle n'est pas relié à l'atome d'oxygène endocyclique,
caractérisé en ce qu'il consiste à faire réagir un alcool de formule R1OH sur l'acide galacturonique de formule: 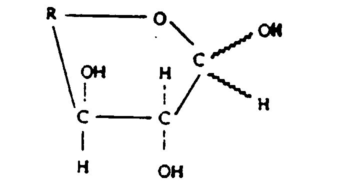 pour obtenir un mélange d'alkylgalactoside uronates d'alkyle de formule 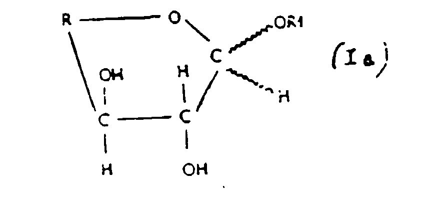 et à séparer chaque alkylgalactoside uronate d'alkyle du mélange.

2. Procédé suivant la revendication 1, caractérisé en ce que R1 est alkyle ayant de 8 à 14 atomes de carbone.

3. Procédé suivant la revendication 2, caractérisé en ce que R1 est décyle.

4. Procédé suivant la revendication 1, caractérisé en ce que R1 est alkyle ayant de 14 à 22 atomes de carbone.

5. L'utilisation d'un dérivé tel que préparé aux revendications 1 à 4 comme agent tensio-actif.

6. L'utilisation d'un dérivé tel que préparé aux revendications 1 à 4 comme détergent.

7. L'utilisation selon la revendication 6, caractérisé en ce que R1 est alkyle ayant de 8 à 14 atomes de carbone.

8. L'utilisation d'un dérivé tel que préparé aux revendications 1 à 4 en cosmétologie.

## Patentansprüche (Patentansprüche für folgende(n) Vertragsstaat(en): AT, BE, CH, DE, DK, FR, GB, GR, IT, LI, LU, MC, NL, PT, SE)

1. Derivat der Galacturonsäure der Formel: 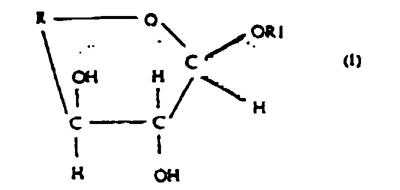 wobei R1 ein lineares oder verzweigtes Alkyl mit 2 bis 22 Kohlenstoffatomen,
R -CH-CH(OH)-CO2R1
oder -CH(OH)-CH-CO2R1 ist, wobei der Kohlenstoff, der die Hydroxylgruppe trägt, nicht an ein Sauerstoffatom des Rings gebunden ist.

2. Derivat nach Anspruch 1, dadurch gekennzeichnet, daß R1 ein Alkyl mit 8 bis 14 Kohlenstoffatomen ist.

3. Derivat nach Anspruch 2. dadurch gekennzeichnet, daß R1 ein aufgespaltener Ring ist.

4. Derivat nach la Anspruch 1, dadurch gekennzeichnet, daß R1 ein Alkyl mit 14 bis 22 Kohlenstoffatomen ist.

5. Verfahren zur Herstellung von Derivaten der Galacturonsäure der Formel (I), dadurch gekennzeichnet, daß es besteht aus:
Umsetzen eines Alkohols der Formel R1OH mit der Galacturonsäure der Formel: 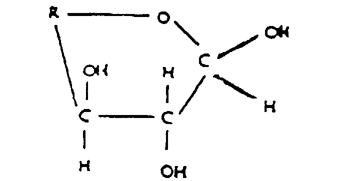 unter Erhalt einer Mischung der Formel: 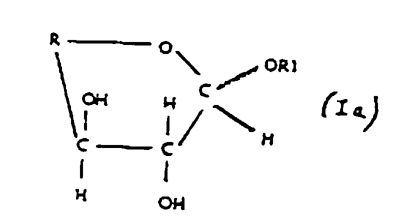 und Abtrennen jedes Alkyl-Galacturonats des Alkyls aus der Mischung.

6. Verwendung eines Derivats, wie in den Ansprüchen 1 bis 5 definiert, als oberflächenaktives Agens.

7. Verwendung eines Derivats, wie in den Ansprüchen 1 bis 5 definiert, als Detergens.

8. Verwendung nach Anspruch 7, dadurch gekenneichnet, daß R1 ein Alkyl mit 8 bis 14 Kohlenstoffatomen ist.

9. Verwendung eines Derivats, wie in den Ansprüchen 1 bis 5 definiert, in der Kosmetik.

## Patentansprüche (Patentansprüche für folgende(n) Vertragsstaat(en): ES)

1. Verfahren zur Herstellung von Derivaten der Galacturonsäure der Formel: 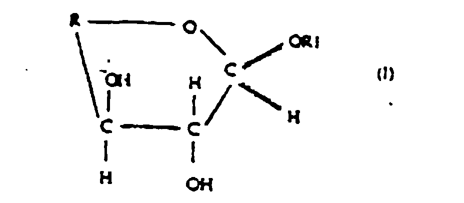 wobei R1 ein geradkettiges oder verzweigtes Alkyl mit 2 bis 22 Kohlenstoffatomen ist,
R 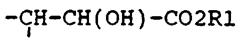 oder 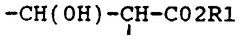 ist, wobei der Kohlenstoff, der die Hydroxylgruppe trägt, nicht mit einem Sauerstoffatom im Ring verbunden ist, gekennzeichnet durch:
Umsetzen eines Alkohols der Formel R1OR mit der Galacturonsäure der Formel: 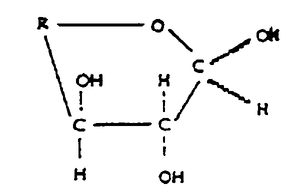 zu einer Mischung von Alkylgalactosiduronaten des Alkyls der Formel 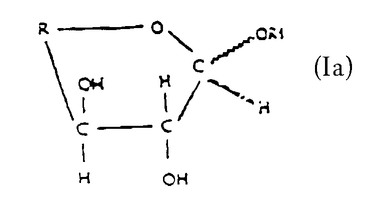 und Abtrennen jedes Alkylgalactosiduronats des Alkyls aus der Mischung.

2. Verfahren nach Anspruch 1, dadurch gekennzeichnet, daß R1 ein Alkyl mit 8 bis 14 Kohlenstoffatomen ist.

3. Verfahren nach Anspruch 2, dadurch gekennzeichnet, daß R1 ein aufgespaltener Ring ist.

4. Verfahren nach Anspruch 1, dadurch gekennzeichnet, daß R1 ein Alkyl mit 14 bis 22 Kohlenstoffatomen ist.

5. Verwendung des nach Ansprüchen 1 bis 4 hergestellten Derivats als oberflächenaktives Mittel.

6. Verwendung des nach Ansprüchen 1 bis 4 hergestellten Derivats als Detergens.

7. Verwendung nach Anspruch 7, dadurch gekennzeichnet, daß R1 ein Alkyl mit 8 bis 14 Kohlenstoffatomen ist.

8. Verwendung eines Derivats wie in Ansprüchen 1 bis 4 hergestellt, in der Kosmetik.

## Claims (Claims for the following Contracting State(s): AT, BE, CH, DE, DK, FR, GB, GR, IT, LI, LU, MC, NL, PT, SE)

1. Galacturonic acid derivatives of formula: 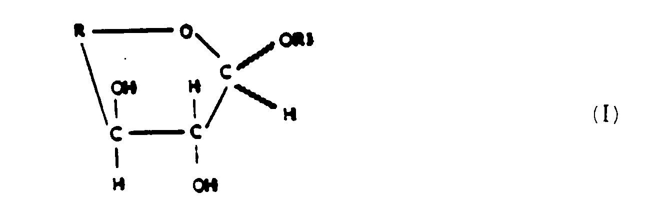
R1 being a linear or branched alkyl having 2 to 22 carbon atoms,
R being:
〉CH-CH(OH)-CO₂R₁
OR 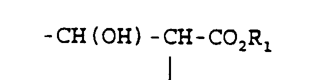 for which the carbon carrying the hydroxyl group is not attached to the endocyclic oxygen atom.

2. Derivatives according to claim 1, characterized in that R1 is an alkyl having 8 to 14 carbon atoms.

3. Derivatives according to claim 2, characterized in that R1 is decyl.

4. Derivatives according to claim 1, characterized in that R1 is an alkyl having 14 to 22 carbon atoms.

5. Process for preparing galacturonic acid derivatives of formula (I), characterized in that it comprises reacting an alcohol of formula R1OH with the galacturonic acid of formula 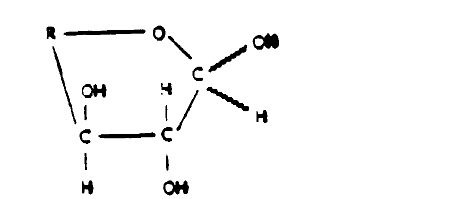 or
to give a mixture of formula 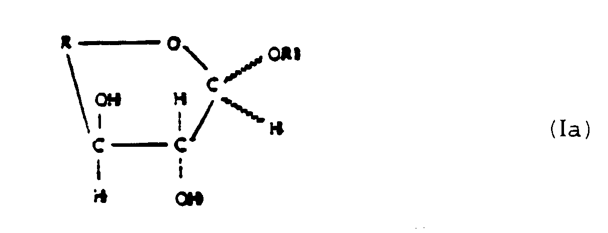 and separating each alkyl alkyl galactoside uronate from the mixture.

6. The use of a derivative as defined in claims 1 to 4 as a surfactant.

7. The use of a derivative as defined in claims 1 to 4 as a detergent.

8. The use according to claim 7, characterized in that R1 is alkyl having 8 to 14 carbon atoms.

9. The use of a derivative as defined in claims 1 to 4 in cosmetology.

## Claims (Claims for the following Contracting State(s): ES)

1. Process for producing galacturonic acid derivatives of formula: 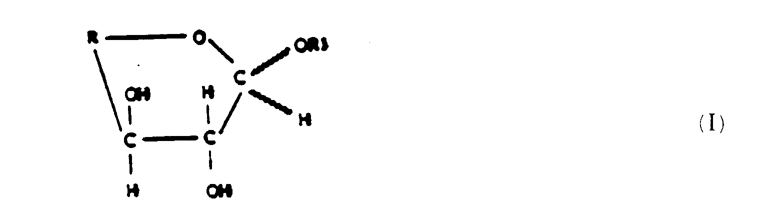
R1 being a linear or branched alkyl having 2 to 22 carbon atoms,
R being:
〉CH-CH(OH)-CO₂R₁
OR 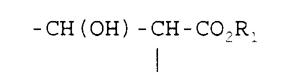 for which the carbon carrying the hydroxyl group is not attached to the endocyclic oxygen atom,
characterized in that it comprises reacting an alcohol of formula R1OH with the galacturonic acid of formula 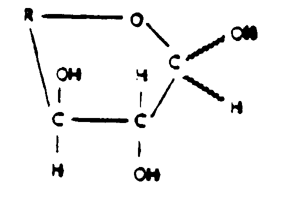 or
to give a mixture of formula 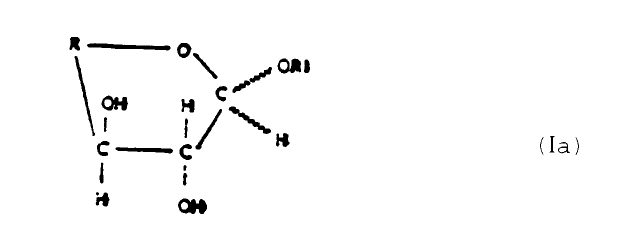 and separating each alkyl alkyl galactoside uronate from the mixture.

2. Process according to claim 1, characterized in that R1 is an alkyl having 8 to 14 carbon atoms.

3. Process according to claim 2, characterized in that R1 is decyl.

4. Process according to claim 1, characterized in that R1 is an alkyl having 14 to 22 carbon atoms.

5. The use of a derivative as prepared in claims 1 to 4 as a surfactant.

6. The use of a derivative as prepared in claims 1 to 4 as a detergent.

7. The use according to claim 7, characterized in that R1 is alkyl having 8 to 14 carbon atoms.

8. The use of a derivative as prepared in claims 1 to 4 in cosmetology.
